# EUROPEAN PATENT APPLICATION

(11) **EP 0 939 122 A1**
(43) Date of publication of application: **01.09.1999**
(21) Application number: 98200621.5
(22) Date of filing: 27.02.1998
(51) Int. Cl.: C12N 15/12, C07K 14/47, C12N 1/19, C12N 5/10, A01K 67/027, C07K 16/18, G01N 33/574, G01N 33/50

(54) **Mouse TCF-3 and TCF-4 and tumorigenesis related to WNT/Wingless signalling**

(71) Applicant: Universiteit Utrecht, 3584 CA Utrecht (NL)
(72) Inventor: The designation of the inventor has not yet been filed
(74) Representative: Smulders, Theodorus A.H.J., Ir.

(57) **Abstract**

The invention relates to the field of diagnosis and treatment of cancer and to Wnt/Wingless signalling. The invention provides an isolated and/or recombinant nucleic acid or a specific fragment, homologue or derivative thereof, corresponding to a mammalian Tcf-3 or Tcf-4 gene with a nucleic acid sequence as shown in figure 9 and encoding a Tcf-3 or Tcf-4 protein, or fragment thereof, said protein comprising a β-catenin binding domain and capable of complexing with β-catenin in response to signalling through the Wnt/Wingless cascade. Furthermore, the invention provides a method for identifying a candidate drug comprising use of a cell comprising a nucleic acid provided by the invention.

## Description

The invention relates to the field of drug discovery, diagnosis and treatment of cancer and to Wnt/Wingless signalling.

Despite extensive knowledge relating to the multitude of cancer forms (varying in appearance from solid tumours and related metastases in distinct parts of the body to leukaemia's of blood cells that circulate throughout the body, and varying from being totally benign to being aggressively malignant) effective therapy of cancer is difficult and in general restricted to three types; treatment via radiation, via chemotherapy and via surgery. Possibilities for a more specific therapy, directed against the underlying cause of a specific cancer or groups of cancers are currently virtually non-existing. Extensive efforts are directed at providing such specific drugs through drug discovery attempts that try to identify candidate drugs for specific cancer therapy.

Development of cancer often starts with changes in a cell that lead to the unrestricted development and division of that first cell into an ever dividing population of cells. These changes are often an accumulation of mutations or other alterations in key genes that occur chronologically, whereby the mutated cell population looses its original, often specialised character and acquires more and more of a cancerous nature. Normal processes of growth regulation of cells are dysfunctioning in the altered cells. Transcription of genes that are normally only little expressed in said cell type is in cancerous cells no longer controlled.

Activation of transcription of genes by transcription factors that would otherwise be dormant in the specific cell type can for example lead to the so typical unrestricted growth and neoplastic nature of cancer. An example are mutations in suppressor genes that function normally by generating proteins that are suppressing transcriptional pathways which are no longer of use in a specialised cell. Mutated suppressor genes do not longer help keep the growth of a cell at bay. Drugs directed against or intervening with the specific protein-protein or protein-DNA interactions in transcriptional pathways controlling cell growth or development can be considered typical candidate drugs for later use in specific cancer therapy, especially when such pathways have gone awry and lead to unrestricted growth cells.

The Wnt/Wingless signalling pathway has been most studied in embryonic development of insects or amphibians where it lies at the basis of broad developmental effects. The Wingless signalling pathway establishes segment polarity in Drosophila, while the ectopic expression of some members of the Wnt signalling cascade induces the axis duplication in Xenopus. A striking symmetry exists between these two pathways (reviewed in 13). Wingless/Wnt signalling is assumed to re-program gene expression in responding cells. Despite the identification of multiple components of this signal transduction pathway, the mechanism by which the reprogramming occurs remains a mystery and until very recently, β-catenin represented the most downstream known component in the Wnt signal transduction pathway (reviewed in 23). β-catenin and its Drosophila homolog armadillo play dual roles in cell-cell adhesion and in signalling. Both proteins occur in adherent junctions in a complex with α-catenin and cadherin homologs, and both accumulate inside cells in response to Wingless/Wnt signals.

The two transcription factors Tcf-1 and Lef-1 are the founding members of a small subfamily of vertebrate HMG box transcription factor genes (5,20,27,28,32). Tcf-1 and Lef-1 were originally defined as lymphoid-specific transcription factors based on their affinity for the enhancers of the CD3ε and the T cell receptor-α genes, respectively. Tcf-1 and Lef-1 were later found to be expressed in largely overlapping, complex patterns during embryogenesis (21). In the course of recent studies, several novel Tcf proteins were cloned from diverse organisms, i.e. dTCF (or Pangolin) from Drosophila (4,29) and XTcf-3 from Xenopus (16).

The biological relevance of the Wnt/Wingless cascade and the interaction between β-catenin and Tcf proteins was tested by ectopic expression of engineered forms of XTcf-3 and Lef-1 in Xenopus embryos. Injection of β-catenin into early Xenopus embryos induces a secondary axis (8), mimicking the broad effect of Wnt activation. Ectopic expression of a dominant-negative form of XTcf-3, lacking the region required for β-catenin binding, blocks β-catenin"s ability to cause axis duplication, and, more importantly, blocks formation of the endogenous axis (16). In concordance with these findings, injection of murine Lef-1 induces the formation of a secondary axis in Xenopus embryos (2,10).

It is tempting to speculate that early Wnt-driven developmental signals during embryogenesis in mammals, such as the mouse, are mediated by transcription factors of the Tcf family. However, the available knockout data for mLef-1 and mTcf-1 are not consistent with a role for these transcription factors in Wnt signalling in the mammalian embryo. To the contrary, gene disruption of Tcf-1 yields a severe but restricted T cell developmental defect as the only phenotypic abnormality (31). Mutation of Lef-1 only results in abnormalities in the development of various skin appendages, teeth and the trigeminal nucleus but leaves the immune system and other organ systems intact (30).

A typical example of a transcriptional pathway gone wrong and leading to development of cancer, can be found with adenomatous polyposis coli (APC). Mutations in this gene are the most common disease-causing events in humans, approximately 50% of the population will, during a normal life span, develop colorectal polyps initiated by APC mutations. Individuals who inherit APC mutations develop thousands of colorectal tumours. APC protein interacts with at least six other proteins, β-catenin, γ-catenin, tubulin, EB1, hDLG and ZW3/GSKβ kinase that may be involved with communicating APC related growth control. Colon carcinoma cells with mutant APC contain large amounts of monomeric, cytoplasmic β-catenin. Reintroduction of wild-type APC reduces the overall amount of β-catenin.

Recently, a human transcription-factor, hTcf-4, was found to be the only member of the Tcf family expressed in colonic epithelium and in the genetically predisposed colorectal polyps found in APC (14) and it was suggested that wild type APC regulates the interaction between β-catenin with hTcf4 whereas mutant APC leads to accumulation of β-catenin. In colon cells, APC therefore regulates the formation of transcriptionally competent β-catenin/Tcf-4 complexes, and loss of APC function results in uncontrolled activation of Tcf-4 target genes, thereby contributing to colon tumourigenesis. This observation was supported by the finding that the constitutive transcriptional activity of Tcf-4-responsive reporter genes was strongly increased in APC ⁻/⁻ colon carcinoma cells but inactive in non-colonic cells or cell lines of tumour or non-tumour origin. Loss of APC function can thus be seen as a crucial event in the early transformation of colonic epithelium.

Above finding of APC related hTcf-4 deregulation is, albeit enhancing insight in tumourigenesis, restricted to the narrow field of colon carcinoma and has therefor only limited value to the field of diagnosis and treatment of cancer as a whole. However, there is an obvious need for gaining understanding and access to diagnosis and treatment in a much broader field.

The invention provides among others access to and insight in protein-protein or protein-DNA interactions in a transcriptional pathway controlling cell growth or development throughout a wide range of cells and tissues of the body, and provides means, such as nucleic acid, protein, cells and experimental animals and methods to identify candidate drugs, for example for use in cancer therapy.

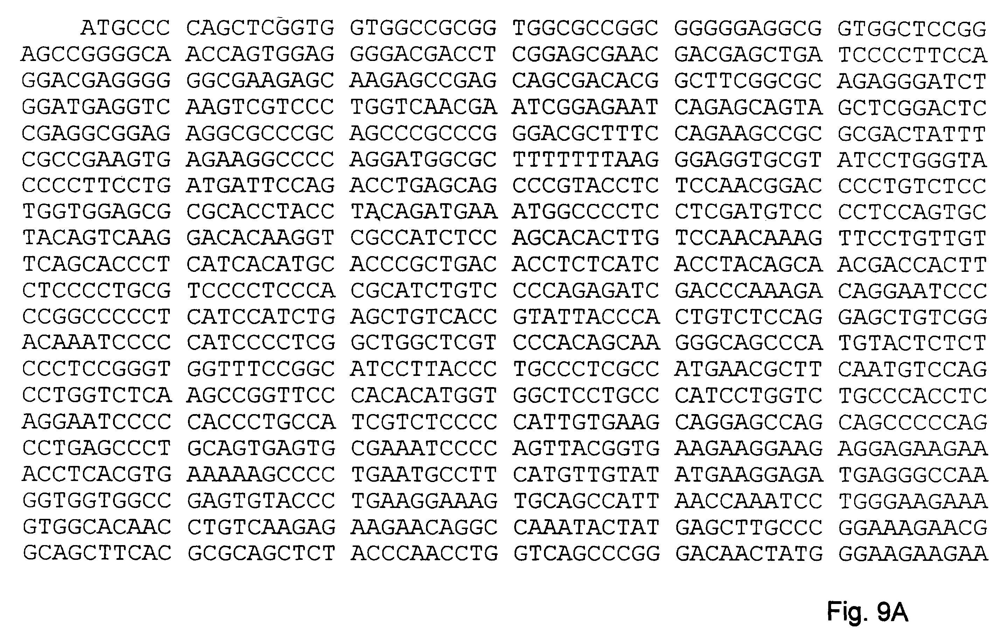

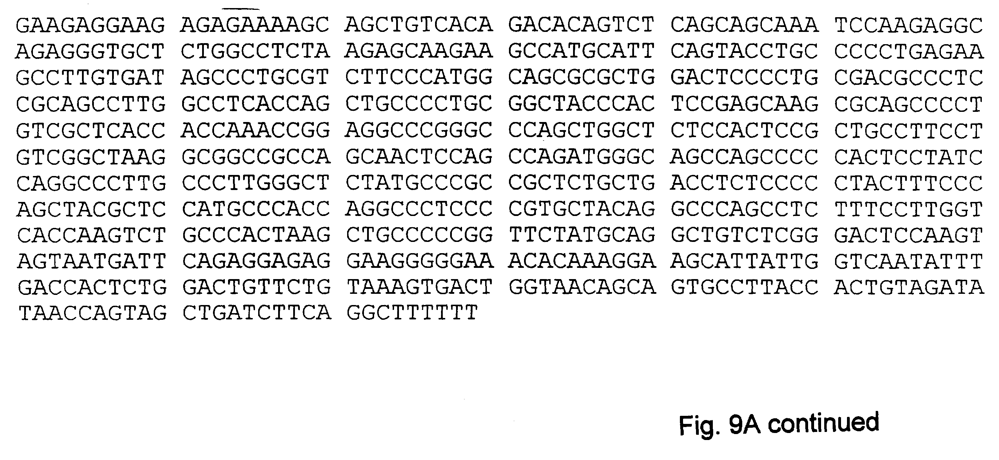

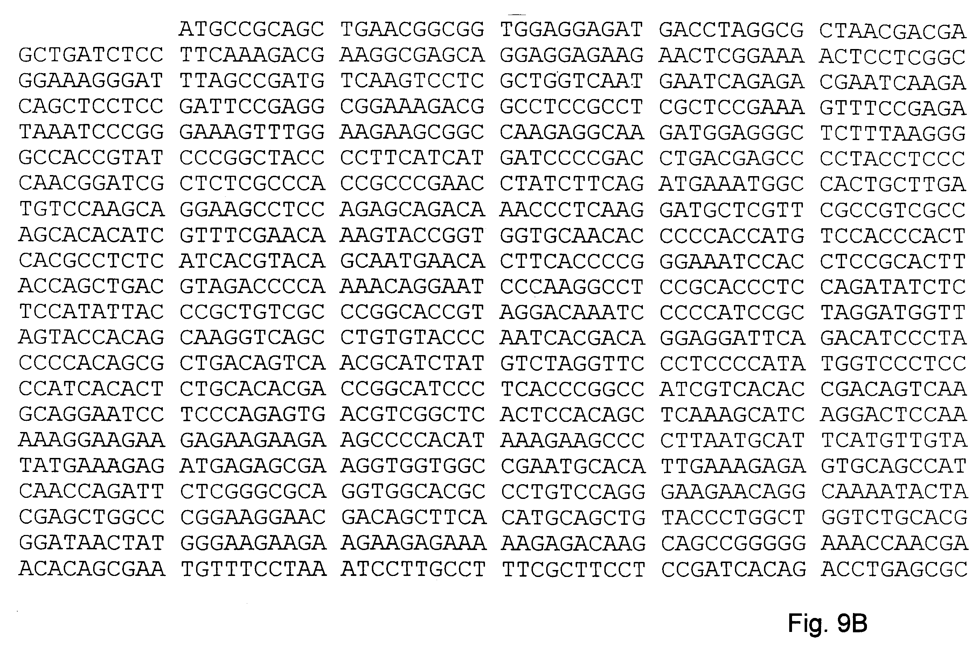

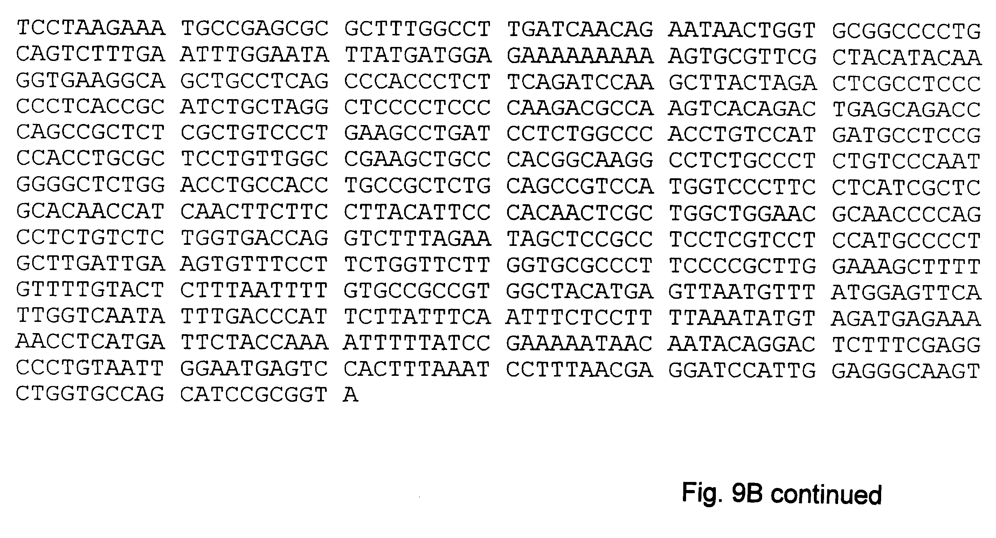

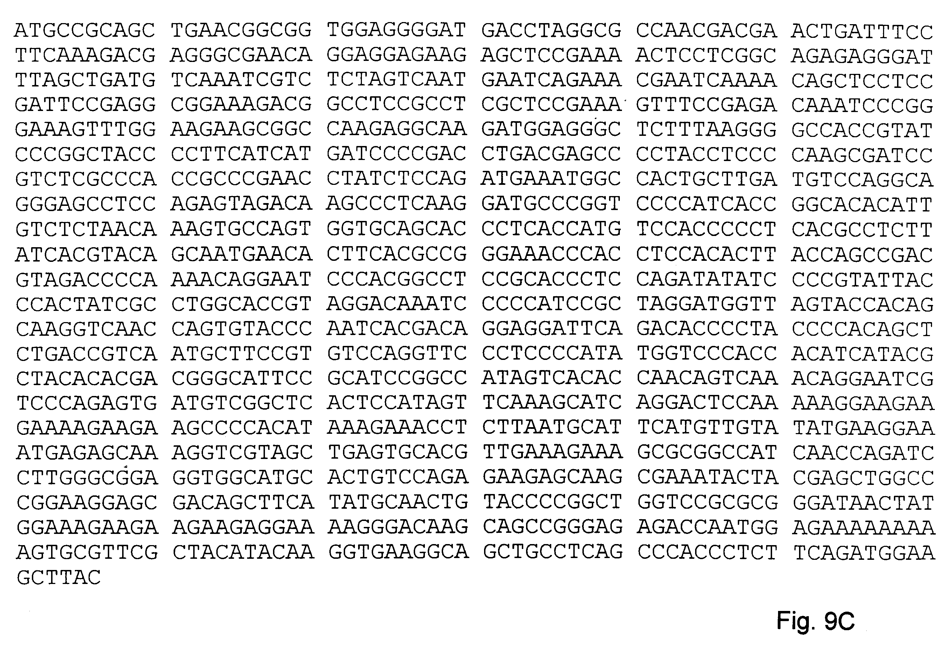

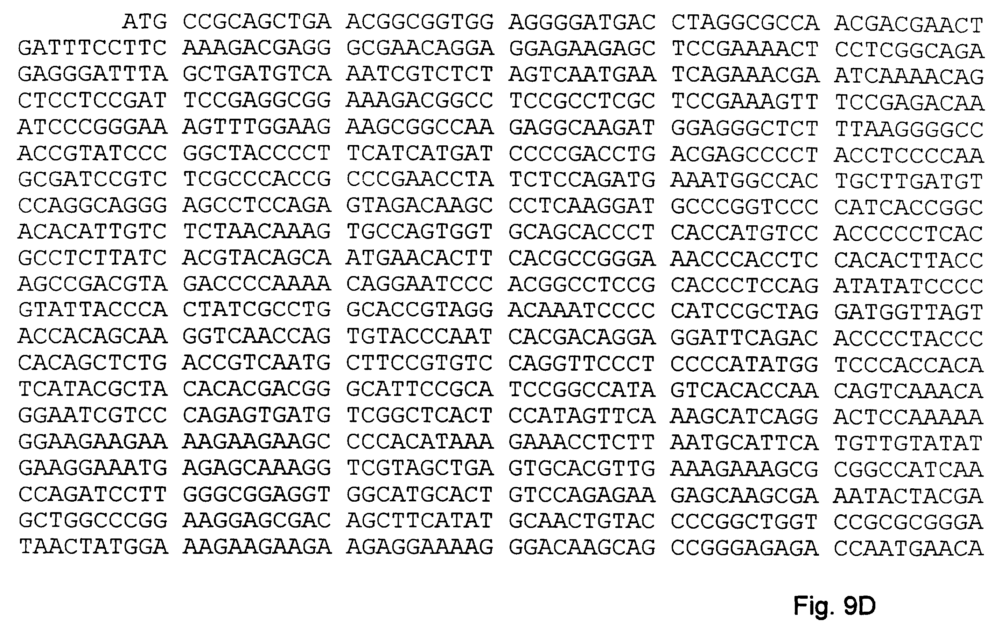

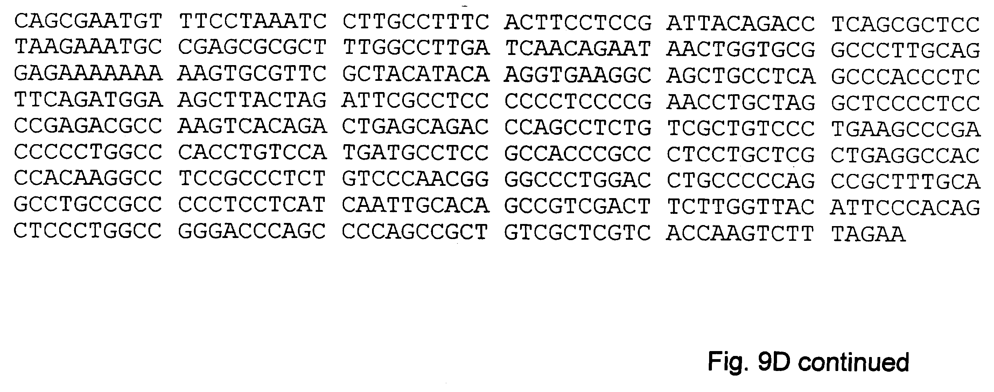

The invention provides an isolated and/or recombinant nucleic acid or a specific fragment, homologue or derivative thereof, corresponding to a mammalian Tcf-3 or Tcf-4 gene with a nucleic acid sequence as shown in figure 9 and encoding a Tcf-3 or Tcf-4 protein or fragment thereof, said protein comprising a β-catenin binding domain and capable of complexing with β-catenin in response to signalling through the Wnt/Wingless cascade. Where in this application the definition "nucleic acid" is used, both RNA and DNA, in single or double-stranded fashion, and nucleic acid hybridising thereto is meant. Examples of specific hybridisation are given in the experimental part, wherein among others specific hybridisation with Tcf-3 or Tcf-4 mRNA in various cells or tissues are shown. "Specific fragment" herein meaning a nucleic acid or part thereof that is functionally or structurally related to or hybridising with a distinct nucleic acid or fragment thereof. Typical examples of such a specific fragment as provided by the invention are the β-catenin binding region, the HMG box, and the regions located N-terminally or C-terminally of the HMG box. Comparing mTcf-3 with mTcf-4 shows that said regions for example have 72, 93, 64 and 20% amino acid identity. "Homologue" herein meaning a related nucleic acid that can be found with another species. "Derivative" herein meaning a nucleic acid that has been derived by genetic modifications, such as deletions, insertions, and mutations from a distinct nucleic acid or fragment thereof. "Corresponding" herein meaning having a nucleic acid sequence homology of at least 80%, more preferably of at least 90%. The invention also provides cDNA, or a specific fragment, homologue or derivative thereof, corresponding to a Tcf-3 or Tcf-4 gene, as for example in the experimental part of the description where cloned cDNAs of these two members of the Tcf family, their expression patterns are determined and their involvement in Wnt signalling is shown.

We have found that Wnt/Wingless signalling regulates the formation of transcriptionally competent β-catenin/Tcf-3 or Tcf-4 complexes, and disturbance of Wnt/Wingless signalling results in uncontrolled activation of Tcf-3 or Tcf-4 target genes, and thus in neoplastic transformation, in a wide variety of cells. The link between Wnt/Wingless signalling and Tcf-3 or Tcf-4 shows that these genes participate in Wnt induced re-programming of gene expression throughout large parts of the organism, also considering the broad patterns of expression seen in the mouse embryo at the various stages of development (see experimental part). Thus, whereas deregulation of APC regulated transcription is limited to hTcf-4 in human colon cells, deregulation of Wnt/Wingless induced transcription of Tcf-3 or Tcf-4 is seen in many cell types of various origin, therewith giving rise to a wide range of neoplasias. Typical examples are foetal cancer, cancers with a stem cell phenotype, testis or ovary teratomas, myeloblastoma, medulloblastoma, mammary or intestinal cancer, and others. Additional cancer types where Wnt/Wingless induced deregulation of transcription of both Tcf-3 or Tcf-4 plays a role can now be identified with a method as provided by the invention, studying Tcf-3 or Tcf-4 transcription activation in cells.

The invention also provides an expression vector comprising a nucleic acid as provided by the invention. An example of such a vector is given in the experimental part of the description, for example as TCF construct mTcf-3: AA 1-321 or TCF construct mTcf-4: AA 1-331. It is within the skills of the artisan to provide vectors that have been provided with single or multiple nucleic acid changes, deletions and/or insertions or other mutations of a nucleic acid sequence.

The invention also provides a cell comprising a genome in which a nucleic acid sequence corresponding to a nucleic acid according to the invention has been modified. Such a modification can comprise a (for example site-directed or transposon-directed or chemically induced) mutation of a nucleic acid encoding a (fragment) of a Tcf-3 or Tcf-4 gene, be it in the intronic or exonic sequences of said gene. The invention also provides a cell comprising a nucleic acid according to the invention that has been introduced via recombinant means known to the skilled artisan, for example via homologous recombination techniques or by using a vector according to the invention. An example of such a cell according to the invention is provided in the experimental part of the description wherein said cell is a yeast cell, such as for use in a two-hybrid mating assay.

The invention also provides a cell capable of expressing a Tcf-3 or Tcf-4 protein or derivative or fragment thereof, said protein comprising a β-catenin binding domain and capable of complexing with β-catenin in response to signalling through the Wnt/Wingless cascade. Such a cell provided by the invention is for example derived of a cell comprising a genome in which a nucleic acid sequence corresponding to a nucleic acid according to the invention has been modified or derived of a cell comprising a nucleic acid according to the invention. Expression of proteins in recombinant cells is in itself a technique available to the average artisan. An example is provided in the experimental part wherein yeast cells expressing a Tcf-3 or Tcf-4 protein are provided.

The invention also provides a conventional cell capable of expressing Tcf-3 or Tcf-4 mRNA or protein or derivative or fragment thereof, said protein comprising a β-catenin binding domain and capable of complexing with β-catenin in response to signalling through the Wnt/Wingless cascade. Cells expressing a specific mRNA can easily be detected by a skilled artisan, for example by applying hybridisation techniques with nucleic acid probes. Similarly, cells expressing a specific protein can be detected by a skilled artisan by applying techniques such as electrophoreses and/or immunological detection. Such a conventional cell as provided by the invention is for example present in a mouse embryo, a preferred example is a stem cell, but is more preferably derived of a cell line, such as an embryonic stem cell line. Other examples of such a cell or cell line expressing a Tcf-3 or Tcf-4 gene product are given in the experimental part, for instance a PC12 cell, a 3T3 fibroblast, a RBL-5 T-lymphocyte or NS-1 B-lymphocyte are provided by the invention. A preferred embodiment of the invention is a cell derived of a mammary epithial cell line C57MG, which is capable of both a high Tcf-3 or Tcf-4 mRNA and protein expression.

The invention also provides a cell capable of expressing a Tcf-3 or Tcf-4 protein or derivative or fragment thereof, said protein comprising a β-catenin binding domain and capable of complexing with β-catenin in response to signalling through the Wnt/Wingless cascade, wherein said cell is additionally provided with a nucleic acid encoding a repressible Wnt gene or derivative or fragment thereof. At least six Wnt family genes (i.e. Wnt-1, -2, -3a, -4, -5a, -5b) are known, which are expressed in largely overlapping regions within the embryo. Null mutants in at least three Wnt genes have been described. An example of a cell encoding a repressible Wnt gene or derivative or fragment thereof provided by the invention can be found in the experimental part of this description wherein a 2-69-23 cell (and a method to obtain such a cell) is provided wherein a repressible Wnt-1 gene has been introduced by recombinant techniques. In a cell (for example a 2-69-23 cell) provided by the invention which is additionally comprising a nucleic acid encoding a repressible Wnt gene or derivative or fragment thereof, Wnt induced Tcf-3 or Tcf-4/β-catenin complexing and subsequent Tcf-target gene trans-activation, and the influence of a variety of compounds thereof, are studied.

The invention also provides a cell capable of expressing a Tcf-3 or Tcf-4 protein or derivative or fragment thereof, said protein comprising a β-catenin binding domain and capable of complexing with β-catenin in response to signalling through the Wnt/Wingless cascade, wherein said cell is additionally provided with a nucleic acid encoding a Tcf-responsive reporter gene. An example of such a cell provided by the invention can be found in the experimental part of this description wherein a 2-69-23 cell is provided wherein a Tcf-reporter gene has been introduced by recombinant techniques. Tcf-responsive reporter (Tcf-reporter) genes are those constructs which comprise a readily detectable or assayable gene (such a luciferase, β-galactosidase, chloramphenicol acetyltransferase) linked in cis to a Tcf-responsive element. Such responsive elements are known in the art and any such elements can be used. An optimal Tcf motif contains the sequence CCTTTGATC. From one to twenty copies, and preferably from three to six copies, of the motif may be used. Mutation of the sequence to CCTTTGGCC abrogates responsiveness. Another necessary part of such constructs is a minimal promotor, such as the c-Fos or the herpesvirus thymidine kinase promotor. Transcription of the reporter gene may be preformed by any means known in the art, usually by assaying for the activity of the encoded gene, although immunological detection methods can also be used. In addition, transcription can be monitored by measuring the transcribed mRNA directly, typically using oligonucleotide probes. In a cell (for example a 2-69-23 cell) provided by the invention which is additionally comprising a nucleic acid encoding a Tcf-reporter gene, Tcf-target gene activation, and the influence of a variety of compounds thereof, are studied by studying the effect of (modifications of ) Tcf or Tcf-complexes and of interactions with compounds therewith, on the transcription of the reporter gene.

The invention also provides an animal comprising a genome in which a nucleic acid sequence corresponding to a Tcf nucleic acid according to the invention has been modified or introduced. Such an animal is for example a transgenic animal obtained by modifying an embryonic stem cell. Stem cell modifications, as well as modifications of other cells, are known to the average skilled artisan. Such an animal is for example a homozygotous knock-out animal (Tcf-3^{-/-} of Tcf-4^{-/-}) or a heterozygotous (Tcf-3^{+/-}of Tcf-4^{+/-}) animal, for example a cross between a knock-out animal with a wild type animal or is otherwise modified in a nucleic acid fragment in its genome that is corresponding to a nucleic acid provided by the invention. A preferred embodiment of the invention is wherein said animal is a mouse.

The invention also provides a Tcf-3 or Tcf-4 protein or derivative or fragment thereof, said protein comprising a β-catenin binding domain and capable of complexing with β-catenin in response to signalling through the Wnt/Wingless cascade. Such a protein or derivative or fragment thereof according to the invention is for example encoded by a nucleic acid according to the invention or produced by a cell according to the invention. Proteins, be it natural proteins or recombinant versions thereof can easily be isolated by a skilled artisan, for example when at least a part of the amino acid sequence, or a specific antibody directed against the protein, is provided. The invention provides such an isolated or recombinant Tcf-3 or Tcf-4 protein or (poly)peptide or derivatives or fragments thereof. In addition, the invention provides an antibody specifically directed against a Tcf-3 or Tcf-4 protein or (poly)peptide or derivative or fragment thereof according the invention. It is within the skills of the average skilled artisan to provide a (synthetic) antibody directed against a protein or fragment thereof, once the amino acid sequence or the isolated protein is provided. An example of such an antibody, which is specifically directed against hTcf4 and mTcf4 is provided in the experimental part of the description.

The invention also provides a method for identifying a candidate drug comprising use of a cell or an animal or a protein or an antibody according to the invention. Candidate drugs, often first selected or generated via combinatorial chemistry, can now be tested and identified using a method provided by the invention. Such a candidate drug or compound can for example be tested on and selected for their effect on Tcf-3 or Tcf-4 regulated transcription, as for example measured by using a Tcf-responsive reporter gene. For example, a test compound or drug which inhibits the transcription of said reporter gene is a candidate drug for cancer therapy. A preferred embodiment of the invention is a method for identifying a candidate drug wherein said drug is tested on β-catenin/Tcf-3 or β-catenin/Tcf-4 regulated transcription, more preferably when said β-catenin/Tcf-3 or β-catenin/Tcf-4 transcription is part of a Wnt/Wingless signalling cascade. For example, a test compound or drug which inhibits the formation of a β-catenin/Tcf-3 or β-catenin/Tcf-4 complex is a candidate drug for cancer therapy. In a most preferred embodiment of the invention, said testing is performed under conditions wherein Wnt/Wingless signalling can be repressed, thereby for example studying the interaction between Wnt/Wingless signalling and a test compound. A method provided by the invention for identifying a candidate drug can be accomplished in vitro or in cells. If the method is to be accomplished in cells, then a Tcf-responsive reporter gene must be introduced into the cell. Any means for introducing genetic material into cells can be used, including but not limited to infection, transfection, electroporation. Suitable cells provided by the invention are cells or cell lines recombinantly or conventionally expressing Tcf-3 or Tcf-4 mRNA or Tcf-3 or Tcf-4 protein. A typical example of such a cell suitable for candidate drug testing is a C57MG cell or cell derived thereof, typically provided with a Tcf-responsive reporter gene and/or with a (repressible) Wnt gene. Yet another typical example is an intestinal cell or cell line wherein for example Tcf-4 expression is found.

A preferred embodiment of the invention is a method for identifying a candidate drug wherein said drug is for use in a cancer patient. In the above it is illustrated that deregulation of transcription is an important cause of cancer. Now that it is possible to study deregulation of transcription-activation by Tcf-3 and Tcf-4 in a wide range of cells under many conditions, for example under various conditions of Wnt-signalling, it is possible to test for drugs that intervene in transcription-activation by Tcf-3 and Tcf-4. Such intervening drugs are extremely useful to treat cancers wherein Wnt/Wingless or Tcf-3 or -4 regulated transcription and re-programming gene expression in cells is an underlying cause of the cancer. An example of such a method provided by the invention is wherein said cancer is related to foetal cancer, cancers with a stem cell phenotype, testis or ovary teratomas, myeloblastoma, medulloblastoma, mammary or intestinal cancer, or wherein such a cancer is associated with Wnt/Wingless signalling or defects therein.

The invention also provides a method for diagnosing cancer comprising use of a nucleic acid or a cell or an animal or a protein according or an antibody provided by the invention. For example cells that express Tcf-3 or Tcf-4 gene products or malignant variants thereof can now easily be detected in samples taken from a patient suspected of having a cancer. The invention is described more in detail in the experimental part of the description without limiting the invention thereto.

### Experimental part

### Cloning of mTcf-3 and mTcf-4

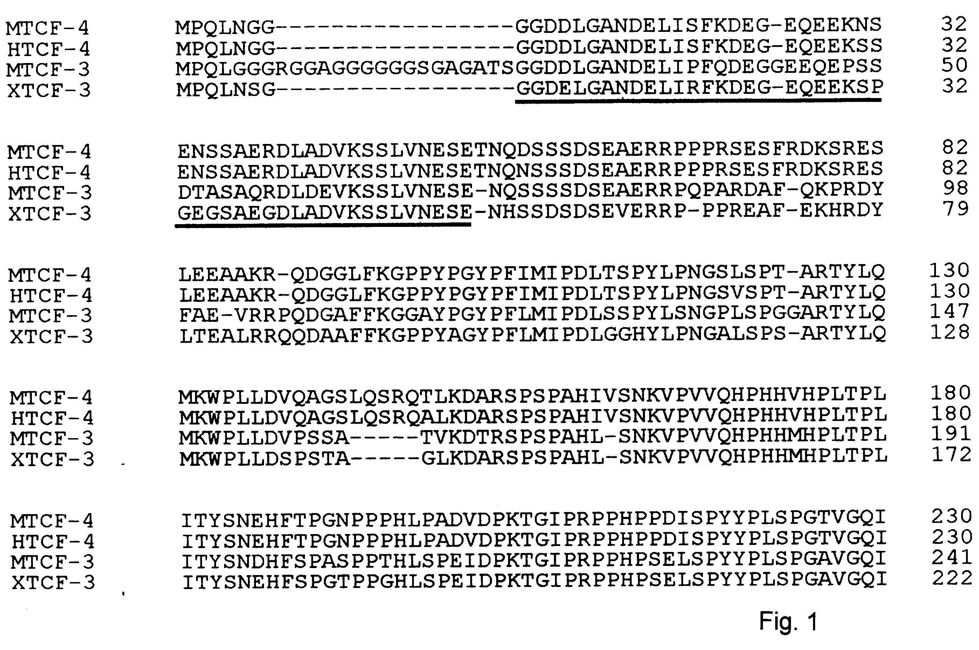

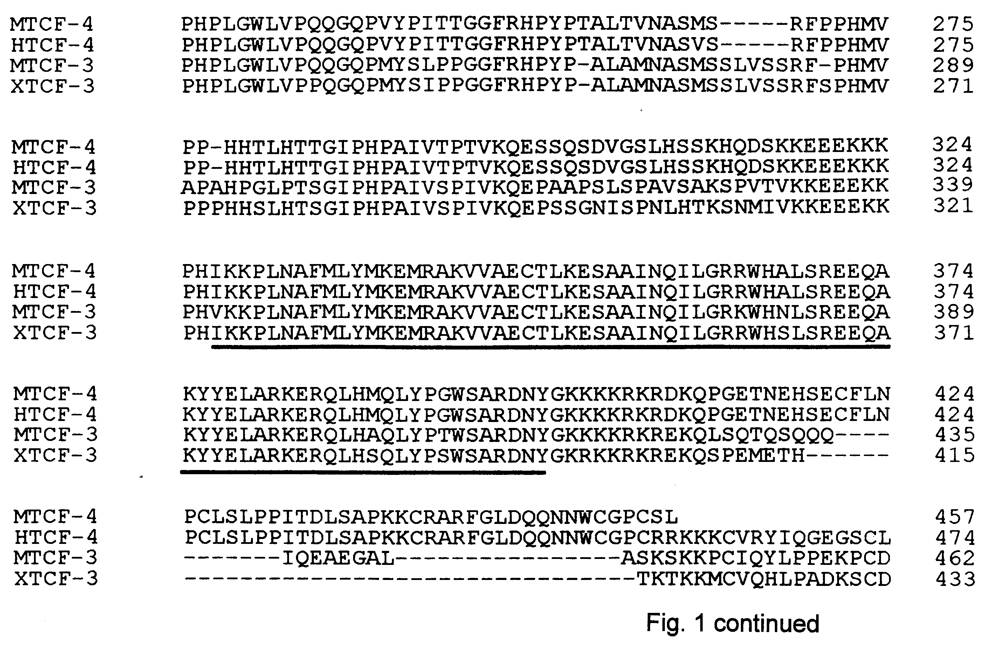

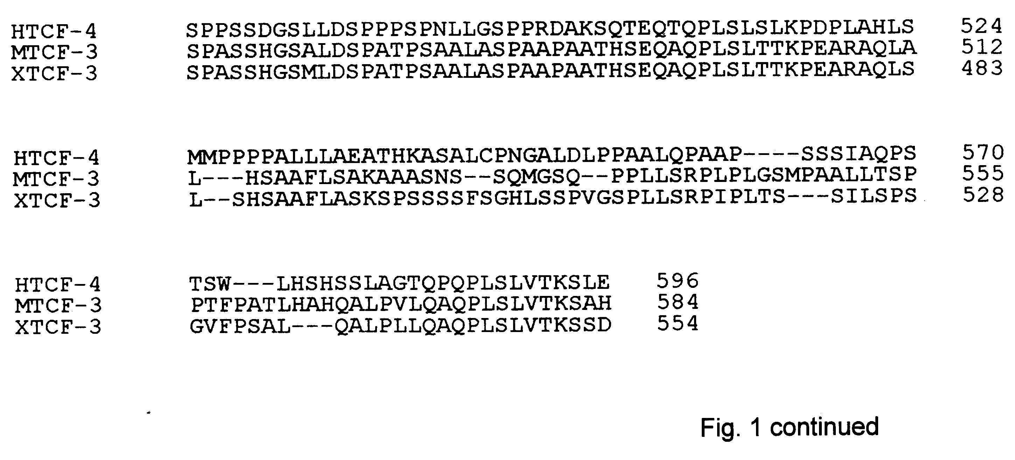

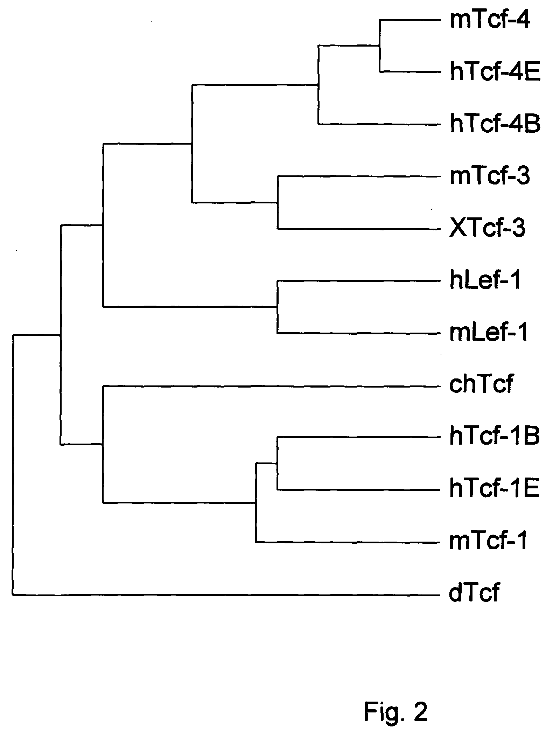

To isolate Tcf-like genes from the mouse, low-stringency screening of several embryonic cDNA libraries was performed with a mixed probe consisting of the HMG boxes of XTcf-3 (16), chTcf (5) and hTcf-1 (28). All efforts yielded cloned sequences derived from two different Tcf genes. The amino acid sequence directly N-terminal to the HMG boxes were aligned with the corresponding regions encoded by the human genomic fragments of hTCF-3 and hTCF-4 (6). We also isolated full length cDNAs for human Tcf-4, which was 98% identical to mTcf-4 at the amino acid level. An alignment of the amino acid sequences of mTcf-3, hTcf-4, mTcf-4 and XTcf-3 is given in Figure 1. An evolutionary tree was constructed for the available Drosophila and vertebrate Tcf sequences using the program Clustal (Figure 2). mTcf-3 clustered with XTcf-3, while mTcf-4 and hTcf-4 clustered separately. The overall match between mTcf-3 and XTcf-3 is 75%. Both proteins align well in the C-terminal regions downstream of the HMG box ( 65% identity), the other three mammalian genes contain less conserved C-termini. The mammalian m- or hTcf-3 and XTcf-3 are encoded by orthologous genes.

Two regions of conservation between mTcf-3, mTcf-4 and other members of the family were of particular interest: 1) The N-terminus, which in XTcf-3, hTcf-1 and -4, Lef-1 and dTCF/Pangolin (2,4,10,14,16,29) constitutes the β-catenin interaction domain, and 2) The HMG box DNA-binding domain, which has >90% identity between individual members of the Tcf family. This predicted that mTcf-3 and -4 would bind to Tcf consensus DNA motifs and would interact with β-catenin.

### Predominantly embryonic expression of mTCF-3 and mTCF-4

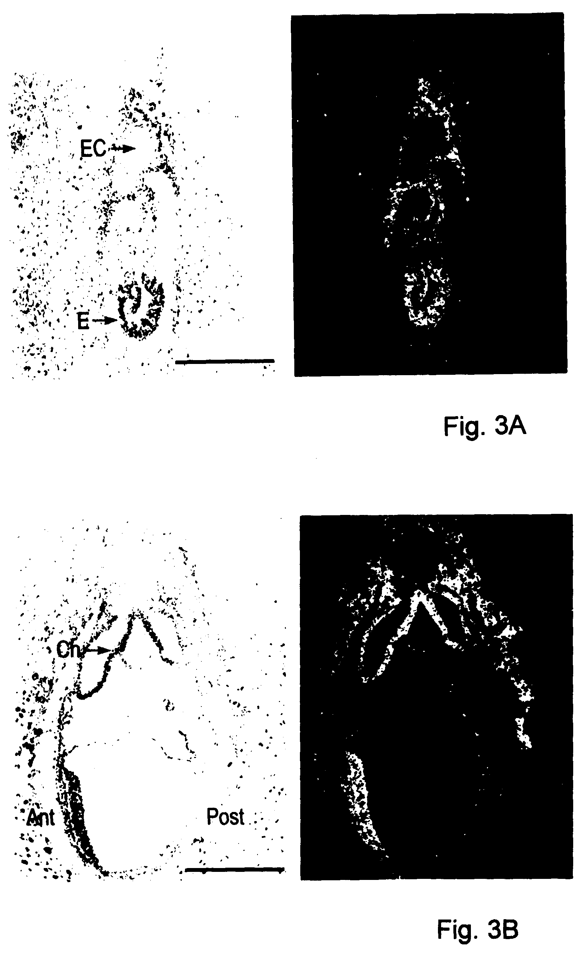

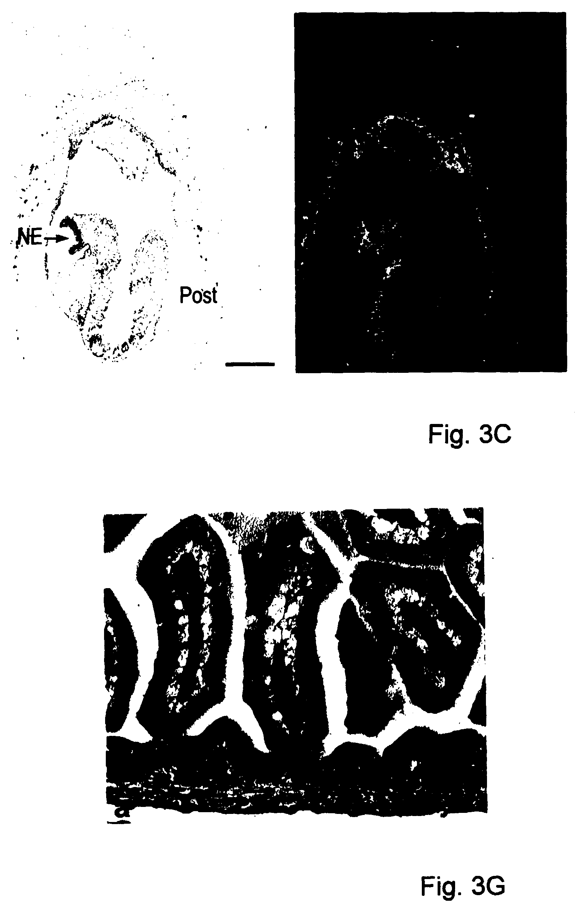

We determined the embryonic expression pattern of mTcf-3 and mTcf-4. As depicted in Figure 3A for a longitudinal section of an E6.5 embryo, strong expression of mTcf-3 was observed throughout the embryo proper. In addition, all extra-embryonic tissues with the exception of the ectoplacental cone and the decidua were positive. By E7.5, expression levels were declining, with highest signals remaining in the anterior part of the embryo. The posterior part, including the primitive streak, showed lower expression levels (Figure 3B). At E8.5, the head region still showed expression, especially in the anterior neurectoderm. Expression had almost disappeared in the posterior part of the embryo (Figure 3C). From day 10.5 onwards, mTcf-3 expression became undetectable (not shown).

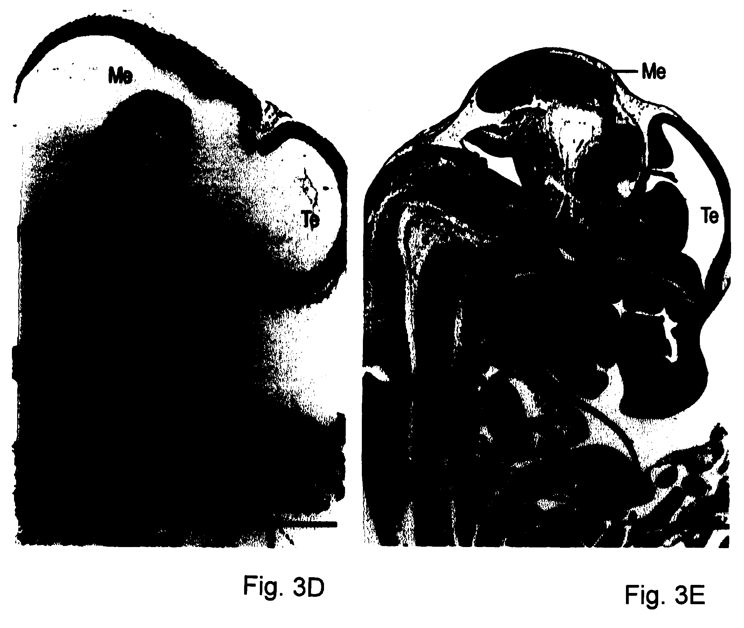

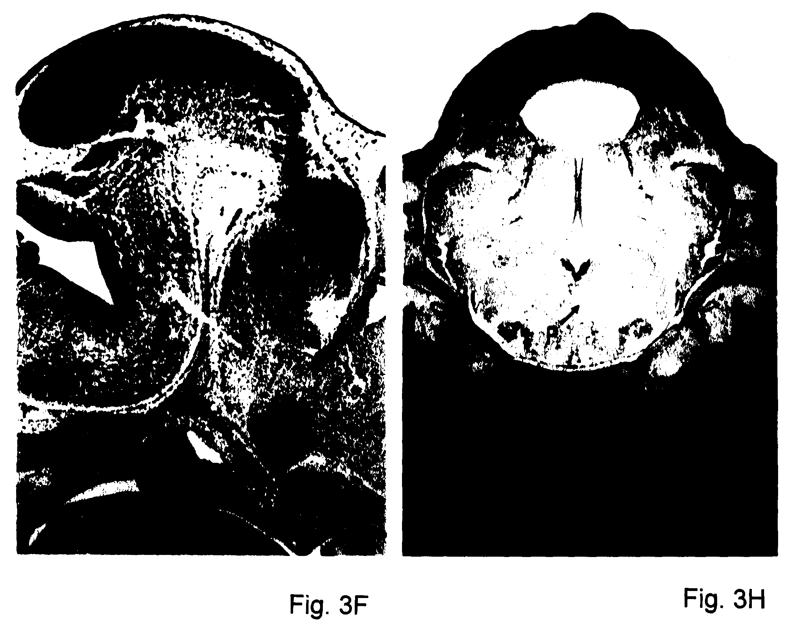

mTcf-4 expression was undetectable on E6.5, E7.5 and E8.5 (not shown), and was first observed at E10.5 in the roof of the diencephalon and in the anterior part of the mesencephalon, with a sharp posterior boundary (Figure 3D). At all later embryonic stages, high-level expression was mostly restricted to the central nervous system. At E13.5, mTcf-4 mRNA was expressed in the roof of the mesencephalon (the tectum) and in the dorsal thalamus. The expression was sharply demarcated from the remainder of the thalamus complex by the mTcf-4-negative zona limitans intrathalamica. High level expression was also detected at the di-telencephalic junction (Figure 3E, 3F). At E13.5, relatively low amount of mTcf-4 protein was observed in the intestinal epithelium (Figure 3G). The intestinal epithelium was the only embryonic tissue with detectable level of mTcf-4 expression outside of the central nervous system. This expression pattern remained essentially unchanged at E16.5. On coronal sections at E16.5, mTcf-4 mRNA was also detected in the pons cerebri (Figure 3H).

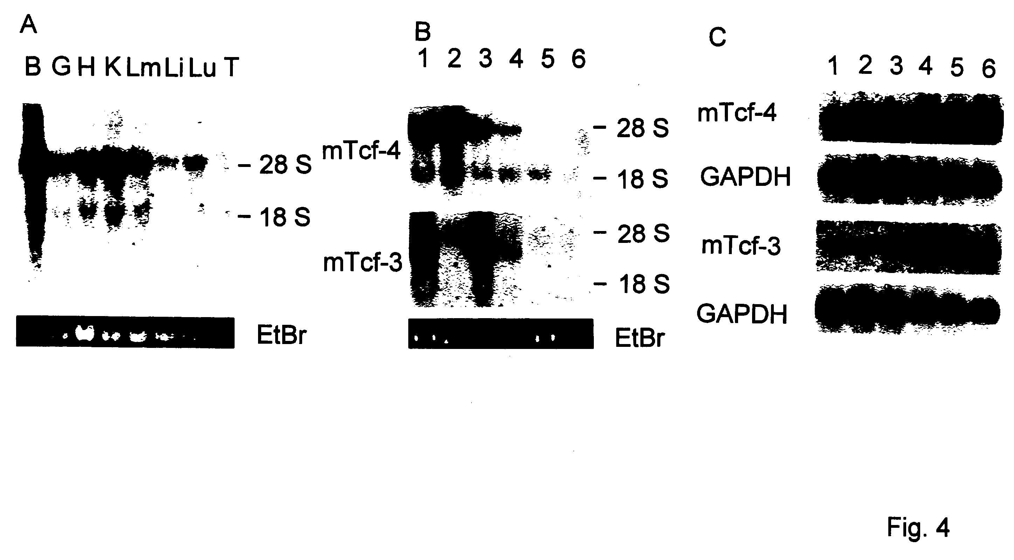

The expression of mTcf-3 and mTcf-4 was also analyzed by Northern blotting on a panel of mouse embryonic (E18.5) and adult tissues, and on several selected cell lines. As expected, no mTcf-3 expression was observed in any of the embryonic or adult tissue RNA samples (not shown). By contrast, high level expression was detected in E14 Embryonic Stem cells, and in the mammary epithelial cell line C57MG, while low level expression occurred in NIH-3T3 fibroblasts (Figure 4B). In line with the in situ data, high level mTcf-4 expression was observed in embryonic brain; all other tissues were essentially negative (Figure 4A), as were all adult tissues tested (not shown). Human Tcf-4 is highly expressed in different cell lines derived from colon adenocarcinomas and hTcf-4 mRNA is clearly detected by in situ analysis in human colon epithelium (14). We noticed a low-level mTcf-4 expression in the intestinal epithelium at E13.5. We realized, that the expression of mTcf-4 in embryonic and adult mouse intestine can not be probably detected by conventional Northern blot using total RNA. We thus performed Northern blot analysis on polyA+ RNA purified from different regions of the mouse intestine. As shown in Figure 4C, mTcf-4 is detected in all parts of the mouse intestine with a clear gradient along the rostro-caudal axis. The relative values indicate a 10-fold increase of mTcf-4 expression in the distal colon compared to the duodenal part of the small intestine. A similar pattern of the expression was detected for mTcf-3 (Figure 4C). In the tested cell lines, high mTcf-4 expression was observed in the C57MG cell line and in the PC12 pheochromocytoma cell line, Embryonic Stem cells and NIH-3T3 fibroblasts express relatively low level of mTcf-4 mRNA (Figure 4B).

### mTcf-3 and mTcf-4 interact with β-catenin.

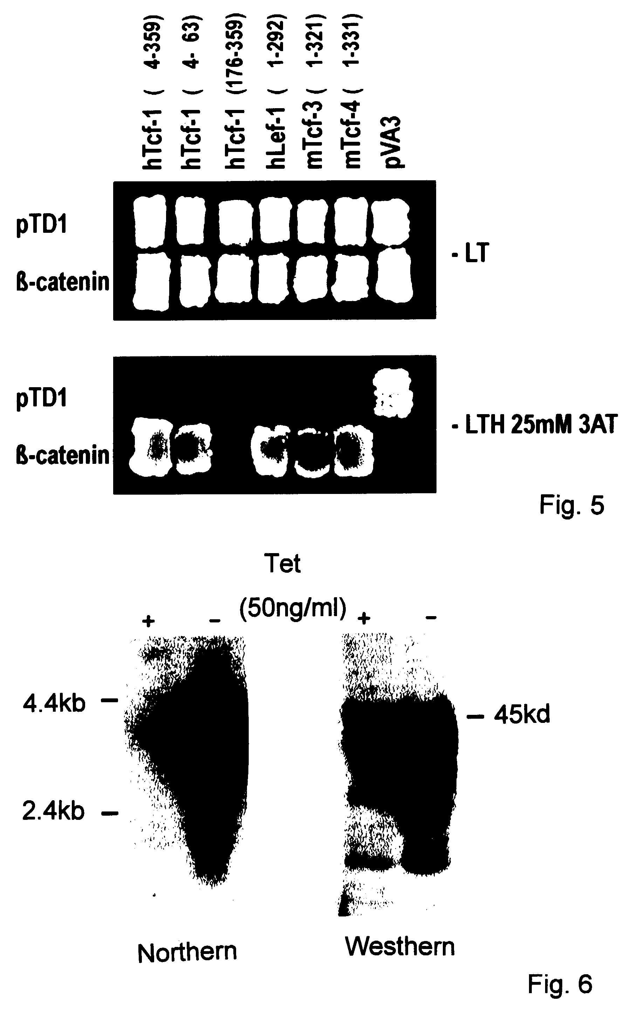

We next wished to obtain molecular support for the involvement of Tcf-3 and Tcf-4 proteins in Wnt/β-catenin signalling. The interaction between other Tcf members and β-catenin occurs between the N-terminus of the former and the Armadillo repeat of the latter. We utilised the yeast two-hybrid system to demonstrate the interaction. As depicted in Figure 5, two-hybrid interactions were readily detectable between the novel Tcf proteins and β-catenin. Wnt-1 expression induces nuclear β-catenin/Tcf complexes and activates.

Up till the present invention no experimental proof has linked Wnt stimulation with the induction of β-catenin/Tcf interactions or with induced Tcf reporter gene transcription. The expression of mTcf-3 and mTcf-4 in the Wnt-1-responsive mammary epithelial C57MG cells, allowed us to establish such a direct link. C57MG cells do not express the Wnt-1 gene (19). Upon stimulation by Wnt-1 protein, C57MG cells show morphological transformation and altered growth characteristics (3,12,33). Transient expression of Wnt-1 in C57MG cells results in the increased stability of β-catenin (22). As shown recently, Wnt-1 stimulation reduces ubiquitination and subsequent degradation of β-catenin via the ubiquitin-proteasome pathway (1). In order to study an effect of the Wnt-1 expression on Tcf/β-catenin mediated transcription we generated C57MG cells carrying the Wnt-1 gene under the control of a tetracycline-repressible promoter. The resulting 2-69-23 cell line shows a strong induction of Wnt-1 mRNA and protein within 24 hours of growth upon removal of tetracycline (Figure 6).

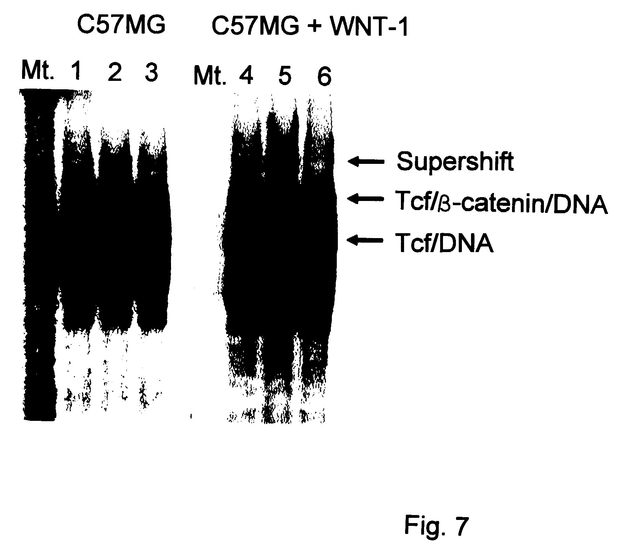

Using a gel retardation/supershift assay, we have previously demonstrated the constitutive presence of nuclear β-catenin/hTcf-4 complex in APC^{-/-} colon carcinoma cells (14) and in colon carcinoma cells with dominant-positive mutations in β-catenin (17). We now applied the same assay to determine whether Wnt stimulation could induce the formation of such complexes. We performed gel retardation with nuclear extracts from 2-69-23 cells stably transfected with a tetracycline-repressible Wnt-1 expression vector. We readily obtained a Tcf/DNA complex from both induced and uninduced cells using an optimal Tcf binding motif as probe. The complex can be supershifted with a monoclonal antibody recognising human and mouse Tcf-4 (Figure 7). The Wnt-1 induction resulted in the appearance of an additional band, which could be further supershifted with a β-catenin antibody (Figure 7). In the repressed state, a much fainter supershifted band with equal properties was visible in these cells, likely reflecting the low level of leakiness of the repression system (Figure 6) . A co-incubation of the binding reaction with both anti β-catenin and anti Tcf-4 antibodies resulted in "super-supershift" confirming simultaneus presence of these proteins in the complex (Figure 7).

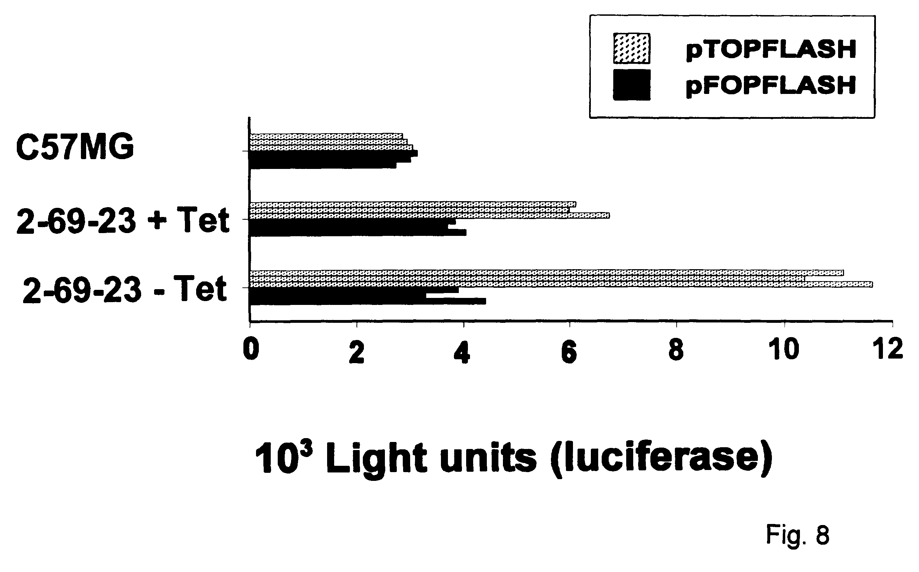

To test if Wnt stimulation could activate transcription from Tcf target genes, we performed transient transfections in C57MG and 2-6923 cells with the Tcf reporter plasmid pTOPFLASH or the mutant negative control plasmid pFOPFLASH (Korinek et al., 1997). In the original C57MG cell line, no differential activities between these two reporters were detected (Figure 8). In 2-69-23 cells, Wnt induction resulted in a three-fold stimulation of the Tcf reporter gene transcription as compared to negative control plasmid. Transfection of 2-69-23 cells growing in a presence of tetracycline already showed about 50% difference between the activities of the reporter constructs (Figure 8). This was consistent with the observed formation of a nuclear β-catenin/Tcf complex upon Wnt stimulation. These data provided formal proof that Wnt stimulation indeed induces the formation of nuclear Tcf/β-catenin complexes and potentially activates transcription from Tcf target genes.

The anti Tcf-4 antibody used for the gel retardation/supershift assay does not crossreact with other mouse Tcf proteins.

### Discussion

The current study describes the characterisation of two members of the Tcf family. Based on their expression profiles and the physical and functional interactions with the Wnt effector β-catenin, Tcf-3 and Tcf-4 mediate Wnt-driven developmental decisions. Taking advantage of the expression of the Tcf genes in Wnt-1 responsive C57MG cells, we provide direct evidence that Wnt-1 stimulation induces the formation of nuclear complexes between Tcf family members and β-catenin, which in turn can activate transcription from Tcf reporter genes.

Based on sequence comparisons, the mTcf-3 cDNA clone is most closely related to the maternally expressed Xenopus Tcf family member, XTcf-3. XTcf-3 is broadly expressed in the Xenopus embryo (16). Like XTcf-3, mTcf-3 is ubiquitously expressed in the gastrulating embryo. From E7.5, mTcf-3 expression is localised to the anterior part of the embryo and then gradually disappears. An earlier study showed an opposite pattern of Tcf-1 and Lef-1 expression with highest levels of both mRNAs in the posterior part of the embryo. Thus, three Tcf family members are expressed in partially overlapping patterns during early postimplantation development.

Expression patterns of mouse Wnt genes have been studied in great detail. Three Wnt family members, Wnt-3a, Wnt-5a and Wnt-5b, are expressed in discrete spatial domains in the primitive streak. The phenotype of Wnt-3a deficient mice indicates that Wnt-3a regulates dorsal mesoderm fate and is required for generation of all embryonic mesoderm. The highest expression of Tcf-1 and Lef-1 in the mouse embryo at E7.5 is observed in embryonic mesoderm and coincides with Wnt-3a expression. This similarity in expressional profile potentially implies Tcf-1/Lef-1 as downstream mediators of Wnt-3a signalling in the early stages of mouse embryogenesis. Based on its expression pattern, we believe that mTcf-3 may have a comparable function in early murine embryogenesis, mediating signalling of a, yet to be identified, Wnt factor in the anterior regions of the gastrulating embryo.

During embryogenesis, mTcf-4 displays a highly specific pattern of expression. It appears much later in development than do the other three family members, and its expression is essentially restricted to the di- and mesencephalon, and the intestinal epithelium. Strikingly, the area of mTcf-4 expression in embryonic brain also expresses high levels of Lef-1 (21,30), suggesting a possible redundancy between Tcf-4 and Lef-1 in midbrain development. At least six Wnt family members are expressed in largely overlapping regions within the central nervous system. Expression patterns of the Wnt-1, -3, -3a genes show a temporal and spatial overlap in di- and mesencephalon with that of Tcf-4 and Lef-1 in this region. Therefore, the latter two Tcf genes are likely candidates to mediate signalling of these Wnt factors. Wnt-3 expression displays a clear boundary between the Wnt-3 positive dorsal thalamus and the Wnt-3 negative remainder of diencephalon. The same sharp boundary of expression was observed for Tcf-4 and Lef-1.

Null mutants in three Wnt genes have been described. It is of obvious interest to compare the phenotypes of Wnt knockout mice with those of Tcf/Lef knockout mice. Only the disruption of the Wnt-1 gene yields a defect in midbrain development. The phenotype of the Wnt-1 and Wnt-3a compound mutant mice has demonstrated clear redundancy of these genes in the regulation of dorsal neural tube differentiation. Despite the suggestion of involvement of Tcf factors in Wingless/Wnt signalling (18), the single knockout phenotypes of Tcf-1 and Lef-1 genes have not implied a role for these two genes in Wnt-1 driven development of the midbrain (15), Wnt-3a regulated somite and tailbud formation, Wnt-4 dependent kidney tubulogenesis or in Wnt-2 regulated development of the placental tissues. The expression patterns of mTcf-3 and mTcf-4 are more restricted in space and time than that of Tcf-1 and Lef-1, and in the case of mTcf-3 implies a specific involvement in early developmental events.

We recently identified hTcf-4 to be expressed in human colonic epithelial cells and cancers derived thereof (14). The avaliable evidence indicates that loss of the tumor-suppressor protein APC or gain-of-function mutations in b-catenin lead to uncontrolled transcriptional activity of Tcf target genes, and -as a consequence- to cellular transformation. The mTcf-4 expression in the embryonic intestinal epithelium suggests a physiological role for this gene in this tissue, possibly in the context of Wnt signalling events that control epithelial homeostasis. In contrast to the brain-specific expression, the intestinal mTcf-4 expression persists to the adult age. mTcf-4 mRNA in mouse intestine is not restricted to the colon, but is also detected in the small intestine. Interestingly, mTcf-4 mRNA shows a gradient with increased levels along the rostro-caudal axis. The same gradient was also detected for mTcf-3.

### LEGENDS TO THE FIGURES.

Figure 1. Sequence comparison of mTcf-4, hTcf-4, mTcf-3, and XTcf-3. The highly conserved N-terminal β-catenin interaction domain and the HMG-box region are underlined.
Figure 2. Phylogenetic relationship among members of the Tcf/Lef gene family based on aligned amino acid sequences. Relative branch lengths are drawn proportional to the number of inferred substitutions per site.
Figure 3. In situ hybridization or immunological analysis of mTcf-3 (A,B,C) and mTcf-4 (D,E,F,G,H) expression on murine embryo sections. mTcf-3 was detected in E6.5 (A), E7.5 (B) and E8.5 (C) embryos. Bright field (left) and the accompanying dark-field (right) pictures of the longitudinal sections are shown. Embryo proper (E), ectoplacental cone (EC), chorion (Ch), neuroectoderm (NE). mTcf-4 expression was first observed at E10.5 (D) in the central nervous system. The expression remains restricted at E13.5 (E). F shows a detail of the head region and G shows a detail of the intestine at E13.5, stained with an antibody directed against Tcf-4. D,E,F and G show parasagittal sections, H shows a coronal section of the head at E16.5. Rhombencephalon (Rh), mesencephalon (Me), diencephalon (Di), telencephalon (Te), tectum (T), dorsal thalamus (DT), di-telencephalic junction (DTJ), (IE) intestinal epithelium, pons (P), esophagus (EP). The horizontal bar in G represents 0.1 mm, in the other sections 0.3 mm.
Figure 4. Northern blot analysis of mTcf-4 and mTcf-3 expression. (A) Tissue specific mTcf-4 expression in mouse embryo at E18.5. The analysis was performed on total RNA isolated from brain (B), gut (G), heart (H), kidney (K), limbs (Lm), liver (Li), lung (Lu), thymus (T). (B) mTcf-4 and mTcf-3 expression in C57MG cells (lane 1), PC12 cells (lane 2), embryonic stem cells (lane 3), 3T3 fibroblasts (line 4), RBL-5 T lymphocyte cells (line 5) and NS-1 B lymphocyte cells (line 6). The positions of 18S and 28S ribosomal RNAs are shown. EtBr: ethidium bromide stain. (C) Northern blot analysis of polyA+ RNA isolated from different parts of intestine. (1) duodenum, (2) jejunum, (3) ileum, (4) caecum, (5) proximal colon, (6) distal colon. GAPDH: control hybridization with GAPDH probe.
Figure 5. Two-hybrid mating assay for the interaction of Tcf"s and β-catenin. Baits ( hTcf-1 (AA 4-359, AA 176-359 and AA4-63), hLef-1 (AA 1-292 ), mTcf-3 (AA 1-321), mTcf-4 (AA 1-331 ), p53) and preys were transformed in MATa or MATa yeast strains respectively, and mated with each other. As shown by growth on selective plates (lower panel) or β-galactosidase assay (not shown), all Tcf"s (but not the N-terminally truncated hTcf-1, or SV40 Large T) interact specifically with β-catenin, and not with a control p53 protein (pVA3). All matings grow on non-selective plates (upper panel).
Figure 6. Inducible expression of Wnt-1 in C57MG cells. (A) Northern blot analysis of Wnt-1 RNA in C57MG cells (clone 2-69-23) carrying a Wnt-1 transgene under the control of a tetracycline (Tet) repressible promoter. PolyA+ RNA was isolated from cells cultured in the presence of Tet (50ng/ml) or for 24 hours in the absence of Tet. The Wnt-1 transcript was detected by blot hybridization after gel electrophoresis using a ³²P-labeled probe for Wnt-1. A longer exposure detects the presence of the major Wnt-1 transcript in Tet treated cells. Wnt-1 expression was quantitated with the Computing Densitometer Model 300A from Molecular Dynamics and basal levels were found to be 50 times lower than the de-repressed levels (data not shown). A probe for Neo was used to confirm the presence of equal levels of RNA in each lane (not shown). (B) Western blot analysis of 2-69-23 cells. Equal amounts of Triton X-100 soluble protein from cells cultured in the presence of Tet (50ng/ml) or for 24 hours in the absence of Tet were analysed by immunoblotting with anti-Wnt-1 antibody.
Figure 7. Wnt-1 induces a Tcf/β-catenin complex in C57MG cells. Gel retardation assay performed on nuclear extracts from 2-69-23 cells growing in presence or absence (Wnt-1 induction) of Tet (50ng/ml). Samples in lines 1 and 5 were incubated under standard conditions. Anti β-catenin antibody (0.2 mg) was added to the samples in lines 2,6 and 9. A control antibody (human CD4, 0.2 mg) was added to the samples in lines 3 and 7. Anti Tcf-4 antibody (affinity purified, 0.2 mg) was added to samples in lines 8 and 9. Samples in lines 4 and 10 were incubated with a probe mutated in the Tcf binding site.
Figure 8. Wnt-1 activates Tcf reporter construct in C57MG cells.
   C57MG cells and 2-69-23 cells growing in the presence or absence of Tet were transfected with 1 mg of the indicated luciferase reporter construct and 1 mg of pCATCONTROL plasmid as internal control. Cells were harvested after 24 hours and luciferase and CAT values were determined.
Figure 9. Nucleotide sequences of mTcf-3 (A), mTcf-4 (B) or hTcf-4 (C and D).

### METHODS

### Cloning of Tcf-3 and Tcf-4 gene

Mouse 11-day and 15-day embryo cDNA libraries (Clontech, Palo Alto, USA) or a human 12-week foetal cDNA library were screened at low stringency with a mixed probe derived from HMG boxes of Tcf. Positive clones were subcloned in pBluescriptSK and sequenced.

### In situ hybridization.

Dissected embryos were fixed 4 to 18 hours in 4% paraformaldehyde, dehydrated in ethanol and butanol and embedded in paraffin. Embryos were sectioned at 6 mm thickness and mounted on 3-aminopropyl-triethoxy-silane coated slides. Slides were deparaffined, digested with proteinase K, acetylated with acetic anhydride dissolved in triethanolamine, dehydrated and hybridized overnight at 55°C. Subsequently, high stringency washing was performed in 50% formamide/2 X SSC/10 mM DTT at 65°C. Slides were treated with ribonuclease A, rinsed in 2 X SSC, 0.1 X SDS, dehydrated and dipped in Ilford emulsion G.5. After drying, the slides were exposed for 1 to 10 days at 4°C, developed in Kodak D-19 and fixed with Kodak LX-24.

### RNA probes

DNA fragments encoding amino acids 270-342 for mTcf-3 and amino acids 258-328 for mTcf-4 were subcloned into pBluescript SK, constructs were linearized and radiolabeled antisense RNA probes were generated from these templates using T7 RNA polymerase and [³⁵S]UTP (Stratagene, La Jolla, CA, USA). Both constructs covered unique DNA sequences. Probes were used for hybridization at 1 x 10⁵ cpm/ml.

### RNA analysis

Total RNA from different tissues and cell lines was isolated according to Chomczynski and Sacchi (7). Approximately 15 mg of total RNA was loaded per line and separated on agarose gel containing 2% formaldehyde. The gel was blotted onto a nitrocellulouse membrane and probed with ³²P- labelled probes. Probes were the same as for the in situ analysis. PolyA+ RNA was isolated from total RNA using Oligo-dT Dynal beads (Dynal, Oslo, Norway). Approximately. 2 mg of polyA+ RNA was loaded per line.

### Tcf-4 antibody

Tcf-4 antibody was raised against the region N-terminal of the HMG box of Tcf-4 in a mouse. It"s specificity was confirmed by screening against COS cells transfected with Tcf-4, Tcf-3, Tcf-1 and Lef-1, and was found positive for human and mouse Tcf-4 only. Staining on mouse and human small intestine shows high levels of Tcf-4 expression in the crypts and villi, with highest levels in the crypts of embryonic small intestine.

### Yeast two hybrid analysis

All TCF constructs were created by fusing the regions encoding the indicated amino acids (AA) to the GAL4 binding domain of pMD4. hTcf-1 constructs: AA 4-359, 4-63 and 176-359. hLef-1: AA 1-292. mTcf-3: AA 1-321. mTcf-4: AA 1-331. pVA3 encodes a murine p53/Gal4 DNA-binding domain hybrid in pGBT9 (Clontech). These constructs were transformed in the MATa yeast strain HF7C (Clontech). β-catenin was inserted in frame with the Gal4 activation domain in pGADrx (Stratagene), pTD1 encodes a SV40 large T-antigen in pGAD3F (Clontech), both were transformed into Y187 (MATa) (Clontech). HF7C cells were mated to Y187 cells. The resulting cells were grown on non-selective (leu⁻/trp⁻), or selective (leu⁻/trp⁻/his⁻/25mM 3-Aminotriazol). β-galactosidase filter assays were performed as described in Matchmaker Two Hybrid System (Clontech).

### Generation of vectors

The vectors for generating cell lines with a tetracycline repressible Wnt-1 cDNA were kindly provided by H. Bujard (9) and modified as follows: pUHD15-1 was digested with BamHI and EcoRI, blunted, to obtain a 1 kb fragment containing the tetracycline sensitive transactivator (tTA). This fragment was cloned into the StuI site of pHyTCX to generate pHyTCtTA (pHyTCX was generated from pLNCX [GenBank name SYNMMLPLN3] by John Murphy who replaced the Neomycin resistance gene with the HygromycinB resistance gene. pUHD10-3 was digested with XhoI and EcoRI to release a 0.46 kb fragment containing a minimal CMV promoter with heptamerized tet-operators. This fragment was cloned into a 6.5 kb EcoRI digested and XhoI partially digested fragment of pROSAbgal (provided by P. Soriano, ref. 25). The resulting vector was named pROTX. An EcoRI fragment containing the Wnt-1 cDNA (from V101) was cloned into the EcoRI site of pROTX to generate pROTWnt-1.

### Generation of 2-69-23 cell line

C57MG cells carrying the Wnt-1 gene under the control of the tetracycline repressible promoter were generated as follows: C57MG cells were first infected with virus obtained from pROTWnt-1 transfected PE501 cells and selected in G418 until colonies appeared. Clones with a flat morphology were subsequently infected with virus obtained from PE501 cells transfected with pHyTCtTA and selected with HygromycinB in the presence of 5 µg/ml tetracycline. Individual colonies were isolated and screened for their ability to induce Wnt-1 overexpression in the absence of tetracycline.

### Wnt-1 Western

Cells were washed in phosphate buffered saline and lysed in ice-cold lysis buffer (1 % Triton X-100, 50 mM Tris-HCl pH8.0, 150 mM NaCl) containing protease inhibitors (1 mM Pefabloc SC [Boehringer Mannheim], 1mM PMSF, 1 ug/ml Leupeptin, 2 ug/ml Aprotinin, 1 ug/ml Pepstatin [Sigma]) and incubated on ice for 20 minutes. Insoluble material was removed by centrifugation at 20,000g for 10 minutes at 4 C. The protein concentration was determined by the method of Bradford (BioRad Protein Assay). Equal amounts of protein were resolved by SDS-polyacrylamide gel electrophoresis and the protein was transferred to nitrocellulose. a-Wnt-1 immunoblots were blocked in 1 % BSA in TBST and incubated in a 1:1000 dilution of the monoclonal α-Wnt-1 antibody Mc123 (raised to a Wnt-1 peptide spanning from amino acid 200 to 212 (3)).

### Cell culture and Wnt-1 induction

C57MG (34) cells were grown in Dulbecco modified Eagle medium (DMEM, Gibco) supplemented with 10% fetal calf serum, 2 mM glutamine, penicillin, streptomycin and 10 mg bovine pancreatic insulin (Sigma) per ml. C57MG cells with a tetracycline-repressible Wnt-1 gene expression (cell line 2-69-23,) were grown in complete medium supplemented with G418 (400 mg/ml, Boehringer), Hygromycin B (100 mg/ml, Boehringer) and tetracycline (50 ng/ml, Sigma). For induction studies, cells were washed with PBS, cultivated for additional 24 hours in tetracycline-free medium and then harvested.

### Gel retardation assays

Assays were performed as described previously (14). Extracts were prepared from intact nuclei of induced and control C57MG cells. Isolated nuclei were washed extensively prior to lysis. The anti β-catenin antibody was purchased from Transduction Laboratories (Lexington, KY). Binding reactions contained 3 mg of nuclear protein, 0.5 ng of probe and 100 ng of poly dIdC in 25 ml of binding buffer (60 mM KCl, 1 mM EDTA, 1 mM DTT, 10% glycerol). Samples were incubated 20 minute at room temperature, antibody was added and samples were incubated for another 20-30 minutes, and subjected to non-denaturing polyacrylamide gel electrophoresis.

### Cat assays and luciferase assays

2*10⁵ C57MG and 2-69-23 cells (induced or noninduced) were transfected with either pTOPFLASH or pFOPFLASH luciferase reporter constructs (Korinek et al., 1997) using LipofectAmine reagent (Gibco). Cells were harvested after 24 hours and lysed in 1 mM DTT, 1 % Triton X-100, 15 % glycerol, 25 mM Tris pH 7.8 and 8 mM MgCl₂. Luciferase activity was determined on a Lumac/3M biocounter. Plasmid pCATCONTROL (Promega) was used as internal control. CAT values were determined as pristane/xylene-extractable radiolabeled, butyrylated chloramphenicol (van de Wetering et al., 1991).

### REFERENCES

1. Aberle, H., Bauer, A., Stappert, J., Kispert, A., and Kemler, R. 1997. β-catenin is a target for the ubiquitin-proteasome pathway. EMBO. J. 16: 3797-3804.
2. Behrens, J., von Kries, J.P., Kuehl, M., Bruhn, L., Wedlich, D., Grosschedl, R., and Birchmeier, W. 1996. Functional interaction of β-catenin with the transcription factor LEF-1. Nature 382: 638-642.
3. Brown, A. M., Wildin, R. S., Prendergast, T. J., and Varmus, H. E. 1986. A retrovirus vector expressing the putative mammary oncogen int-1 causes partial transformation of a mammary epithelial cell line. Cell 46: 1001-1009.
4. Brunner, E., Peter, O., Schweizer, L., and Basler, K. 1997. Pangolin encodes a Lef-1 homologue that acts downstream of Armadillo to transduce the Wingless signal in Drosophila. Nature 385: 829-833.
5. Castrop, J., Hoevenagel, R., Young, J.R., and Clevers, H.C. 1992a. A common ancestor of the mammalian transcription factors TCF-1 and TCF-1a/LEF-1 expressed in chicken T cells. Eur. J. Immunol. 22: 327-1330.
6. Castrop, J., van Norren, K., and Clevers, H.C. 1992b. A gene family of HMG box factors with homology to TCF-1. Nucleic. Acids Res. 20: 611.
7. Chomczynski, P., and Sacchi, N. 1987. Single-Step of RNA Isolation by Acid Guanidinium Thiocyanate-Phenol-Chloroform Extraction. Anal. Biochem. 162: 156-159.
8. Funayama, N., Fagotto, F., McCrea, P., and Gumbiner, B.M. 1995. Embryonic axis induction by the armadillo repeat domain of b-catenin: evidence for intracellular signalling. J. Cell. Biol. 128: 959-968.
9. Gossen, M. , and Bujard, H. 1992. Tight control of gene expression in mammalian cells by tetracycline-responsive promoters. Proc. Natl. Acad.Sci. USA 89: 5547-51.
10. Huber, O., Korn, R., McLaughlin, J., Ohsugi, M., Herrmann B.G., and Kemler, R. 1996. Nuclear localization of β-catenin by interaction with transcription factor LEF-1. Mech. Dev. 59: 3-10.
11. Joyner, A.L. 1996. Engrailed, Wnt and Pax genes regulate midbrain-hindbrain development. Trends Genet. 12: 15-20.
12. Jue, S.F., Bradley, R.S, Rudnicki, J.A., Varmus, H.E., and Brown, A.M. 1992. The mouse Wnt-1 gene can act via a paracrine mechanism in transformation of mammary epithelial cells. Mol.Cell.Biol. 12: 321-8.
13. Klymkowsky, M.W., and Parr, B. 1995. The body language of cells: the intimate connection between cell adhesion and behavior. Cell 83: 5-8.
14. Korinek, V, Barker, N., Morin, P.J., van Wichen, D., de Weger, R., Kinzler, K.W., Vogelstein, B., and Clevers, H. 1997. Constitutive Transcriptional Activation by a β-catenin-Tcf complex in APC^{-/-} Colon Carcinoma. Science 275: 1784-1787.
15. McMahon, A.P. and Bradley, A. 1990. The Wnt-1 (int-1) proto-oncogene is required for development of a large region of the mouse brain. Cell 62: 1073-1085.
16. Molenaar, M, van de Wetering, M., Oosterwegel, M., Peterson-Maduro, J., Godsave, S., Korinek, V., Roose, J., Destree, O., and Clevers, H. 1996. XTcf-3 transcription factor mediates β-catenin-induced axis formation in Xenopus embryos. Cell 86: 391-399.
17. Morin, P.J., Sparks, A., Korinek, V., Barker, N., Clevers, H., Vogelstein, B., and Kinzler, K. 1997. Activation of β-catenin-Tcf signalling in colon cancer by mutations in β-catenin or APC. Science 275: 1787-1790.
18. Nusse, R. 1997. A versatile transcriptional effector of Wingless signalling. Cell 89: 321-323.
19. Olson, D.J., and Papkoff, J. 1994. Regulated expression of Wnt family members during proliferation of C57mg mammary cells. Cell. Growth. Differ. 5: 197-206.
20. Oosterwegel, M., van de Wetering, M., Dooijes, D., Klompr, L., Winotor, A., Georgopoulos, K., Meijlink, F., and Clevers, H. 1991. Cloning of murine TCF-1, a T cell-specific transcription factor interacting with functional motifs in the CD3-e and T cell receptor α enhancers. J. Exp. Med. 173: 1133-1142.
21. Oosterwegel M, van de Wetering, M., Timmerman, J., Kruisbeek, A., Destree, O., Meijlink, F., and Clevers, H. 1993. Differential expression of the HMG box factors TCF-1 and LEF-1. Development 118: 439-448.
22. Papkoff, J., Rubinfeld, B., Schryver, B., and Polakis, P. 1996. Wnt-1 regulates free pools of catenins and stabilizes APC-catenin complexes. Mol. Cell. Biol. 16: 2128-2134.
23. Peifer, M. 1995. Cell adhesion and signal transduction: The Armadillo connection. Trends Cell. Biol. 5: 224-229.
24. Riese, J., Yu, X., Munnerlyn, A., Eresh, S., Hsu, S.-C., Grosschedl, R., and Bienz, M. 1997. LEF-1, a nuclear factor coordinating signalling imputs from wingless and decapentaplegic. Cell 88: 777-787.
25. Soriano, P., Friedrich, G., and Lawinger, P. 1991. Promoter interactions in retrovirus vectors introduced into fibroblasts and embryonic stem cells. J. Virol. 65: 2314-19.
26. Thomas, K.R., and Capecchi, M.R. 1990. Targeted disruption of the murine int-1 proto-oncogene resulting in severe abnormalities in midbrain and cerebellar development. Nature 346: 847-850.
27. Travis, A., Amsterdam, A., Belanger, C., and Grosschedl, R. 1991. Lef-1, a gene encoding a lymphoid-specific protein with an HMG domain, regulates T-cell receptor a enhancer function. Genes Dev. 5: 880-894.
28. van de Wetering, M., Oosterwegel, M., Dooijes, D., and Clevers, H. 1991. Identification and cloning of TCF-1, a T cell-specific transcription factor containing a sequence-specific HMG box. EMBO J. 10: 123-132.
29. van de Wetering, M., Cavallo, R., Dooijes, D., van Beest, M., van Es, J., Loureiro, J., Ypma, A., Hursh, D., Jones, T., Bejsovec, A., Peifer, M., Mortin, M., and Clevers, H. 1997. Armadillo co-activates transcription driven by the product of the Drosophila segment polarity gene dTCF. Cell 88: 789-799.
30. van Genderen, C., Okamura, R.M., Farinas, I., Quo, R.G., Parslow, T.G., Bruhn, L. and Grosschedl, R. 1994. Development of several organs that require inductive epithelial-mesenchymal interactions is impaired in LEF-1 deficient mice. Genes Dev. 8: 2691-2704.
31. Verbeek, S., Izon, D., Hofhuis, F., Robanus-Maandag, E., te Riele, H., van de Wetering, M., Oosterwegel, M., Wilson, A., MacDonald, H.R., and Clevers, H. 1995. An HMG-box-containing T-cell factor required for thymocyte differentiation. Nature 374: 70-74.
32. Waterman, M.L., Fischer, W.H., and Jones, K.A. 1991. A thymus-specific member of the HMG protein family regulates the human T cell receptor Ca enhancer. Genes Dev. 5: 656-669.
33. Wong, G.T., Gavin, B.J., and McMahon, A.P. 1994. Differential transformation of mammary epithelial cells by Wnt genes. Mol.Cell.Biol. 14: 6278-86.
34. Vaida et al., 1978. Virology 90:12-12

## Claims

1. An isolated and/or recombinant nucleic acid or a specific fragment, homologue or derivative thereof, corresponding to a mammalian Tcf-3 or Tcf-4 gene with a nucleic acid sequence as shown in figure 9 and encoding a Tcf-3 or Tcf-4 protein, or fragment thereof, said protein comprising a β-catenin binding domain and capable of complexing with β-catenin in response to signalling through the Wnt/Wingless cascade.

2. A nucleic acid according to claim 1 which is a cDNA molecule.

3. A nucleic acid according to claim 1 or 2 which is of mouse origin.

4. An expression vector comprising a nucleic acid according to anyone of claims 1 to 3.

5. A cell comprising a genome in which a nucleic acid sequence corresponding to a nucleic acid according to anyone of claims 1 to 3 has been modified.

6. A cell comprising a nucleic acid according to anyone of claims 1 to 3 or a vector according to claim 4.

7. A cell according to claim 6 wherein said cell is a yeast cell.

8. A cell capable of expressing a Tcf-3 or Tcf-4 protein or fragment thereof, said protein comprising a β-catenin binding domain and capable of complexing with β-catenin in response to signalling through the Wnt/Wingless cascade.

9. A cell according to claim 8, said cell derived of a cell according to any of claims 5 to 7.

10. A cell according to claim 8, said cell derived of a cell line.

11. A cell according to claim 10, said cell derived of a mammary epithelial cell line C57MG.

12. A cell according to any of claims 8 to 11, additionally provided with a nucleic acid encoding a repressible Wnt gene or derivative or fragment thereof and/or provided with a nucleic acid encoding a Tcf-responsive reporter gene.

13. An animal comprising a cell according to claim 5 or 6.

14. A Tcf-3 or Tcf-4 protein or derivative or fragment thereof, said protein comprising a β-catenin binding domain and capable of complexing with β-catenin in response to signalling through the Wnt/Wingless cascade.

15. A protein or derivative or fragment thereof according to claim 14 encoded by a nucleic acid according to anyone of claims 1 to 3 or produced by a cell according to anyone of claims 5 to 12.

16. An antibody specifically directed against a protein or derivative or fragment thereof according to claim 14 or 15.

17. A method for identifying a candidate drug comprising use of a cell according to any of claims 5 to 12 or an animal according to claim 13 or a protein according to claim 14 or 15 or an antibody according to claim 16.

18. A method according to claim 17 wherein said drug is for use in a cancer patient.

19. A method according to claim 18 wherein said cancer is related to foetal cancer, cancers with a stem cell phenotype, testis or ovary teratomas, myeloblastoma, medulloblastoma, mammary or intestinal cancer.

20. A method for diagnosing cancer comprising use of a nucleic acid according to anyone of claims 1 to 3, a cell according to any of claims 5 to 12 or an animal according to claim 13 or a protein according to claim 14 or 15 or an antibody according to claim 16.


SEQUENCE LISTING

(1) GENERAL INFORMATION:

(i) APPLICANT:
(A) NAME: Universiteit Utrecht
(B) STREET: Sorbonnelaan 16
(C) CITY: Utrecht
(D) STATE: Utrecht
(E) COUNTRY: the Netherlands
(F) POSTAL CODE (ZIP): 3584 CA

(ii) TITLE OF INVENTION: Tumourigenesis related to Wnt/Wingless
signalling.

(iii) NUMBER OF SEQUENCES: 10

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98200621.5

(2) INFORMATION FOR SEQ ID NO: 1:

(i) SEQUENCE CHARACTERISTICS:
(A) LENGTH: 9 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 1:

CCTTTGATC 9

(2) INFORMATION FOR SEQ ID NO: 2:

(i) SEQUENCE CHARACTERISTICS:

(A) LENGTH: 9 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 2:

CCTTTGGCC 9

(2) INFORMATION FOR SEQ ID NO: 3:

(i) SEQUENCE CHARACTERISTICS:
(A) LENGTH: 459 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 3:

Met Pro Gln Leu Asn Gly Gly Gly Gly Asp Asp Leu Gly Ala Asn Asp
1               5                   10                  15

Glu Leu Ile Ser Phe Lys Asp Glu Gly Glu Gln Glu Glu Lys Asn Ser
            20                  25                  30

Glu Asn Ser Ser Ala Glu Arg Asp Leu Ala Asp Val Lys Ser Ser Leu
        35                  40                  45

Val Asn Glu Ser Glu Thr Asn Gln Asp Ser Ser Ser Asp Ser Glu Ala
    50                  55                  60

Glu Arg Arg Pro Pro Pro Arg Ser Glu Ser Phe Arg Asp Lys Ser Arg
65                  70                  75                  80

Glu Ser Leu Glu Glu Ala Ala Lys Arg Gln Asp Gly Gly Leu Phe Lys

                85                  90                  95

Gly Pro Pro Tyr Pro Gly Tyr Pro Phe Ile Met Ile Pro Asp Leu Thr
            100                 105                 110

Ser Pro Tyr Leu Pro Asn Gly Ser Leu Ser Pro Thr Ala Arg Thr Tyr
        115                 120                 125

Leu Gln Met Lys Trp Pro Leu Leu Asp Val Gln Ala Gly Ser Leu Gln
    130                 135                 140

Ser Arg Gln Thr Leu Lys Asp Ala Arg Ser Pro Ser Pro Ala His Ile
145                 150                 155                 160

Val Ser Asn Lys Val Pro Val Val Gln His Pro His His Val His Pro
                165                 170                 175

Leu Thr Pro Leu Ile Thr Tyr Ser Asn Glu His Phe Thr Pro Gly Asn
            180                 185                 190

Pro Pro Pro His Leu Pro Ala Asp Val Asp Pro Lys Thr Gly Ile Pro
        195                 200                 205

Arg Pro Pro His Pro Pro Asp Ile Ser Pro Tyr Tyr Pro Leu Ser Pro
    210                 215                 220

Gly Thr Val Gly Gln Ile Pro His Pro Leu Gly Trp Leu Val Pro Gln
225                 230                 235                 240

Gln Gly Gln Pro Val Tyr Pro Ile Thr Thr Gly Gly Phe Arg His Pro
                245                 250                 255

Tyr Pro Thr Ala Leu Thr Val Asn Ala Ser Met Ser Arg Phe Pro Pro
            260                 265                 270

His Met Val Pro Pro His His Thr Leu His Thr Thr Gly Ile Pro His
        275                 280                 285

Pro Ala Ile Val Thr Pro Thr Val Lys Gln Glu Ser Ser Gln Ser Asp
    290                 295                 300

Val Gly Ser Leu His Ser Ser Lys His Gln Asp Ser Lys Lys Glu Glu
305                 310                 315                 320

Glu Lys Lys Lys Pro His Ile Lys Lys Pro Leu Asn Ala Phe Met Leu
                325                 330                 335

Tyr Met Lys Glu Met Arg Ala Lys Val Val Ala Glu Cys Thr Leu Lys
            340                 345                 350

Glu Ser Ala Ala Ile Asn Gln Ile Leu Gly Arg Arg Trp His Ala Leu
        355                 360                 365

Ser Arg Glu Glu Gln Ala Lys Tyr Tyr Glu Leu Ala Arg Lys Glu Arg
    370                 375                 380

Gln Leu His Met Gln Leu Tyr Pro Gly Trp Ser Ala Arg Asp Asn Tyr
385                 390                 395                 400

Gly Lys Lys Lys Lys Arg Lys Arg Asp Lys Gln Pro Gly Glu Thr Asn
                405                 410                 415

Glu His Ser Glu Cys Phe Leu Asn Pro Cys Leu Ser Leu Pro Pro Ile
            420                 425                 430

Thr Asp Leu Ser Ala Pro Lys Lys Cys Arg Ala Arg Phe Gly Leu Asp
        435                 440                 445

Gln Gln Asn Asn Trp Cys Gly Pro Cys Ser Leu
    450                 455

(2) INFORMATION FOR SEQ ID NO: 4:

(i) SEQUENCE CHARACTERISTICS:
(A) LENGTH: 596 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 4:

Met Pro Gln Leu Asn Gly Gly Gly Gly Asp Asp Leu Gly Ala Asn Asp
1               5                   10                  15

Glu Leu Ile Ser Phe Lys Asp Glu Gly Glu Gln Glu Glu Lys Ser Ser
            20                  25                  30

Glu Asn Ser Ser Ala Glu Arg Asp Leu Ala Asp Val Lys Ser Ser Leu
        35                  40                  45

Val Asn Glu Ser Glu Thr Asn Gln Asn Ser Ser Ser Asp Ser Glu Ala

50                  55                  60

Glu Arg Arg Pro Pro Pro Arg Ser Glu Ser Phe Arg Asp Lys Ser Arg
65                  70                  75                  80

Glu Ser Leu Glu Glu Ala Ala Lys Arg Gln Asp Gly Gly Leu Phe Lys
                85                  90                  95

Gly Pro Pro Tyr Pro Gly Tyr Pro Phe Ile Met Ile Pro Asp Leu Thr
            100                 105                 110

Ser Pro Tyr Leu Pro Asn Gly Ser Val Ser Pro Thr Ala Arg Thr Tyr
        115                 120                 125

Leu Gln Met Lys Trp Pro Leu Leu Asp Val Gln Ala Gly Ser Leu Gln
    130                 135                 140

Ser Arg Gln Ala Leu Lys Asp Ala Arg Ser Pro Ser Pro Ala His Ile
145                 150                 155                 160

Val Ser Asn Lys Val Pro Val Val Gln His Pro His His Val His Pro
                165                 170                 175

Leu Thr Pro Leu Ile Thr Tyr Ser Asn Glu His Phe Thr Pro Gly Asn
            180                 185                 190

Pro Pro Pro His Leu Pro Ala Asp Val Asp Pro Lys Thr Gly Ile Pro
        195                 200                 205

Arg Pro Pro His Pro Pro Asp Ile Ser Pro Tyr Tyr Pro Leu Ser Pro
    210                 215                 220

Gly Thr Val Gly Gln Ile Pro His Pro Leu Gly Trp Leu Val Pro Gln
225                 230                 235                 240

Gln Gly Gln Pro Val Tyr Pro Ile Thr Thr Gly Gly Phe Arg His Pro
                245                 250                 255

Tyr Pro Thr Ala Leu Thr Val Asn Ala Ser Val Ser Arg Phe Pro Pro
            260                 265                 270

His Met Val Pro Pro His His Thr Leu His Thr Thr Gly Ile Pro His
        275                 280                 285

Pro Ala Ile Val Thr Pro Thr Val Lys Gln Glu Ser Ser Gln Ser Asp
    290                 295                 300

Val Gly Ser Leu His Ser Ser Lys His Gln Asp Ser Lys Lys Glu Glu
305                 310                 315                 320

Glu Lys Lys Lys Pro His Ile Lys Lys Pro Leu Asn Ala Phe Met Leu
                325                 330                 335

Tyr Met Lys Glu Met Arg Ala Lys Val Val Ala Glu Cys Thr Leu Lys
            340                 345                 350

Glu Ser Ala Ala Ile Asn Gln Ile Leu Gly Arg Arg Trp His Ala Leu
        355                 360                 365

Ser Arg Glu Glu Gln Ala Lys Tyr Tyr Glu Leu Ala Arg Lys Glu Arg
    370                 375                 380

Gln Leu His Met Gln Leu Tyr Pro Gly Trp Ser Ala Arg Asp Asn Tyr
385                 390                 395                 400

Gly Lys Lys Lys Lys Arg Lys Arg Asp Lys Gln Pro Gly Glu Thr Asn
                405                 410                 415

Glu His Ser Glu Cys Phe Leu Asn Pro Cys Leu Ser Leu Pro Pro Ile
            420                 425                 430

Thr Asp Leu Ser Ala Pro Lys Lys Cys Arg Ala Arg Phe Gly Leu Asp
        435                 440                 445

Gln Gln Asn Asn Trp Cys Gly Pro Cys Arg Arg Lys Lys Lys Cys Val
    450                 455                 460

Arg Tyr Ile Gln Gly Glu Gly Ser Cys Leu Ser Pro Pro Ser Ser Asp
465                 470                 475                 480

Gly Ser Leu Leu Asp Ser Pro Pro Pro Ser Pro Asn Leu Leu Gly Ser
                485                 490                 495

Pro Pro Arg Asp Ala Lys Ser Gln Thr Glu Gln Thr Gln Pro Leu Ser
            500                 505                 510

Leu Ser Leu Lys Pro Asp Pro Leu Ala His Leu Ser Met Met Pro Pro
        515                 520                 525

Pro Pro Ala Leu Leu Leu Ala Glu Ala Thr His Lys Ala Ser Ala Leu
    530                 535                 540

Cys Pro Asn Gly Ala Leu Asp Leu Pro Pro Ala Ala Leu Gln Pro Ala
545                 550                 555                 560

Ala Pro Ser Ser Ser Ile Ala Gln Pro Ser Thr Ser Trp Leu His Ser
                565                 570                 575

His Ser Ser Leu Ala Gly Thr Gln Pro Gln Pro Leu Ser Leu Val Thr
            580                 585                 590

Lys Ser Leu Glu
        595

(2) INFORMATION FOR SEQ ID NO: 5:

(i) SEQUENCE CHARACTERISTICS:
(A) LENGTH: 584 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 5:

Met Pro Gln Leu Asn Gly Gly Arg Gly Gly Ala Gly Gly Gly Gly Gly
1               5                   10                  15

Gly Ser Gly Ala Gly Ala Thr Ser Gly Gly Asp Asp Leu Gly Ala Asn
            20                  25                  30

Asp Glu Leu Ile Pro Phe Gln Asp Glu Gly Gly Glu Glu Gln Glu Pro
        35                  40                  45

Ser Ser Asp Thr Ala Ser Ala Gln Arg Asp Leu Asp Glu Val Lys Ser
    50                  55                  60

Ser Leu Val Asn Glu Ser Glu Asn Gln Ser Ser Ser Ser Asp Ser Glu
65                  70                  75                  80

Ala Glu Arg Arg Pro Gln Pro Ala Arg Asp Ala Phe Gln Lys Pro Arg
                85                  90                  95

Asp Tyr Phe Ala Glu Val Arg Arg Pro Gln Asp Gly Ala Phe Phe Lys
            100                 105                 110

Gly Gly Ala Tyr Pro Gly Tyr Pro Phe Leu Met Ile Pro Asp Leu Ser
        115                 120                 125

Ser Pro Tyr Leu Ser Asn Gly Pro Leu Ser Pro Gly Gly Ala Arg Thr
    130                 135                 140

Tyr Leu Gln Met Lys Trp Pro Leu Leu Asp Val Pro Ser Ser Ala Thr
145                 150                 155                 160

Val Lys Asp Thr Arg Ser Pro Ser Pro Ala His Leu Ser Asn Lys Val
                165                 170                 175

Pro Val Val Gln His Pro His His Met His Pro Leu Thr Pro Leu Ile
            180                 185                 190

Thr Tyr Ser Asn Asp His Phe Ser Pro Ala Ser Pro Pro Thr His Leu
        195                 200                 205

Ser Pro Glu Ile Asp Pro Lys Thr Gly Ile Pro Arg Pro Pro His Pro
    210                 215                 220

Pro Glu Leu Ser Pro Tyr Tyr Pro Leu Ser Pro Gly Ala Val Gly Gln
225                 230                 235                 240

Ile Pro His Pro Leu Gly Trp Leu Val Pro Gln Gln Gly Gln Pro Met
                245                 250                 255

Tyr Ser Leu Pro Pro Gly Gly Phe Arg His Pro Tyr Pro Ala Leu Ala
            260                 265                 270

Met Asn Ala Ser Met Ser Ser Leu Val Ser Ser Arg Phe Pro His Met
        275                 280                 285

Val Ala Pro Ala His Pro Gly Leu Pro Thr Ser Gly Ile Pro His Pro
    290                 295                 300

Ala Ile Val Ser Pro Ile Val Lys Gln Glu Pro Ala Ala Pro Ser Leu
305                 310                 315                 320

Ser Pro Ala Val Ser Ala Lys Ser Pro Val Thr Val Lys Lys Glu Glu
                325                 330                 335

Glu Lys Lys Pro His Val Lys Lys Pro Leu Asn Ala Phe Met Leu Tyr
            340                 345                 350

Met Lys Glu Met Arg Ala Lys Val Val Ala Glu Cys Thr Leu Lys Glu
        355                 360                 365

Ser Ala Ala Ile Asn Gln Ile Leu Gly Arg Lys Trp His Asn Leu Ser
    370                 375                 380

Arg Glu Glu Gln Ala Lys Tyr Tyr Glu Leu Ala Arg Lys Glu Arg Gln
385                 390                 395                 400

Leu His Ala Gln Leu Tyr Pro Thr Trp Ser Ala Arg Asp Asn Tyr Gly
                405                 410                 415

Lys Lys Lys Lys Arg Lys Arg Glu Lys Gln Leu Ser Gln Thr Gln Ser
            420                 425                 430

Gln Gln Gln Ile Gln Glu Ala Glu Gly Ala Leu Ala Ser Lys Ser Lys
        435                 440                 445

Lys Pro Cys Ile Gln Tyr Leu Pro Pro Glu Lys Pro Cys Asp Ser Pro
    450                 455                 460

Ala Ser Ser His Gly Ser Ala Leu Asp Ser Pro Ala Thr Pro Ser Ala
465                 470                 475                 480

Ala Leu Ala Ser Pro Ala Ala Pro Ala Ala Thr His Ser Glu Gln Ala
                485                 490                 495

Gln Pro Leu Ser Leu Thr Thr Lys Pro Glu Ala Arg Ala Gln Leu Ala
            500                 505                 510

Leu His Ser Ala Ala Phe Leu Ser Ala Lys Ala Ala Ala Ser Asn Ser
        515                 520                 525

Ser Gln Met Gly Ser Gln Pro Pro Leu Leu Ser Arg Pro Leu Pro Leu
    530                 535                 540

Gly Ser Met Pro Ala Ala Leu Leu Thr Ser Pro Pro Thr Phe Pro Ala
545                 550                 555                 560

Thr Leu His Ala His Gln Ala Leu Pro Val Leu Gln Ala Gln Pro Leu
                565                 570                 575

Ser Leu Val Thr Lys Ser Ala His
            580

(2) INFORMATION FOR SEQ ID NO: 6:

(i) SEQUENCE CHARACTERISTICS:
(A) LENGTH: 554 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 6:

Met Pro Gln Leu Asn Ser Gly Gly Gly Asp Glu Leu Gly Ala Asn Asp
1               5                   10                  15

Glu Leu Ile Arg Phe Lys Asp Glu Gly Glu Gln Glu Glu Lys Ser Pro
            20                  25                  30

Gly Glu Gly Ser Ala Glu Gly Asp Leu Ala Asp Val Lys Ser Ser Leu
        35                  40                  45

Val Asn Glu Ser Glu Asn His Ser Ser Asp Ser Asp Ser Glu Val Glu
    50                  55                  60

Arg Arg Pro Pro Pro Arg Glu Ala Phe Glu Lys His Arg Asp Tyr Leu
65                  70                  75                  80

Thr Glu Ala Leu Arg Arg Gln Gln Asp Ala Ala Phe Phe Lys Gly Pro
                85                  90                  95

Pro Tyr Ala Gly Tyr Pro Phe Leu Met Ile Pro Asp Leu Gly Gly His
            100                 105                 110

Tyr Leu Pro Asn Gly Ala Leu Ser Pro Ser Ala Arg Thr Tyr Leu Gln
        115                 120                 125

Met Lys Trp Pro Leu Leu Asp Ser Pro Ser Thr Ala Gly Leu Lys Asp
    130                 135                 140

Ala Arg Ser Pro Ser Pro Ala His Leu Ser Asn Lys Val Pro Val Val
145                 150                 155                 160

Gln His Pro His His Met His Pro Leu Thr Pro Leu Ile Thr Tyr Ser
                165                 170                 175

Asn Glu His Phe Ser Pro Gly Thr Pro Pro Gly His Leu Ser Pro Glu
            180                 185                 190

Ile Asp Pro Lys Thr Gly Ile Pro Arg Pro Pro His Pro Pro Glu Leu
        195                 200                 205

Ser Pro Tyr Tyr Pro Leu Ser Pro Gly Ala Val Gly Gln Ile Pro His
    210                 215                 220

Pro Leu Gly Trp Leu Val Pro Pro Gln Gly Gln Pro Met Tyr Ser Ile
225                 230                 235                 240

Pro Pro Gly Gly Phe Arg His Pro Tyr Pro Ala Leu Ala Met Asn Ala
                245                 250                 255

Ser Met Ser Ser Leu Val Ser Ser Arg Phe Ser Pro His Met Val Pro
            260                 265                 270

Pro Pro His His Ser Leu His Thr Ser Gly Ile Pro His Pro Ala Ile
        275                 280                 285

Val Ser Pro Ile Val Lys Gln Glu Pro Ser Ser Gly Asn Ile Ser Pro
    290                 295                 300

Asn Leu His Thr Lys Ser Asn Met Ile Val Lys Lys Glu Glu Glu Lys
305                 310                 315                 320

Lys Pro His Ile Lys Lys Pro Leu Asn Ala Phe Met Leu Tyr Met Lys
                325                 330                 335

Glu Met Arg Ala Lys Val Val Ala Glu Cys Thr Leu Lys Glu Ser Ala
            340                 345                 350

Ala Ile Asn Gln Ile Leu Gly Arg Lys Trp His Ser Leu Ser Arg Glu
        355                 360                 365

Glu Gln Ala Lys Tyr Tyr Glu Leu Ala Arg Lys Glu Arg Gln Leu His
    370                 375                 380

Ser Gln Leu Tyr Pro Ser Trp Ser Ala Arg Asp Asn Tyr Gly Lys Arg
385                 390                 395                 400

Lys Lys Arg Lys Arg Glu Lys Gln Ser Pro Glu Met Glu Thr His Thr
                405                 410                 415

Lys Thr Lys Lys Met Cys Val Gln His Leu Pro Ala Asp Lys Ser Cys
            420                 425                 430

Asp Ser Pro Ala Ser Ser His Gly Ser Met Leu Asp Ser Pro Ala Thr
        435                 440                 445

Pro Ser Ala Ala Leu Ala Ser Pro Ala Ala Pro Ala Ala Thr His Ser
    450                 455                 460

Glu Gln Ala Gln Pro Leu Ser Leu Thr Thr Lys Pro Glu Ala Arg Ala
465                 470                 475                 480

Gln Leu Ser Leu Ser His Ser Ala Ala Phe Leu Ala Ser Lys Ser Pro
                485                 490                 495

Ser Ser Ser Ser Phe Ser Gly His Leu Ser Ser Pro Val Gly Ser Pro

            500                 505                 510

Leu Leu Ser Arg Pro Ile Pro Leu Thr Ser Ser Ile Leu Ser Pro Ser
        515                 520                 525

Gly Val Phe Pro Ser Ala Leu Gln Ala Leu Pro Leu Leu Gln Ala Gln
    530                 535                 540

Pro Leu Ser Leu Val Thr Lys Ser Ser Asp
545                 550

(2) INFORMATION FOR SEQ ID NO: 7:

(i) SEQUENCE CHARACTERISTICS:
(A) LENGTH: 1945 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 7:

ATGCCCCAGC TCGGTGGTGG CCGCGGTGGC GCCGGCGGGG
GAGGCGGTGG CTCCGGAGCC 60

GGGGCAACCA GTGGAGGGGA CGACCTCGGA GCGAACGACG
AGCTGATCCC CTTCCAGGAC 120

GAGGGGGGCG AAGAGCAAGA GCCGAGCAGC GACACGGCTT
CGGCGCAGAG GGATCTGGAT 180

GAGGTCAAGT CGTCCCTGGT CAACGAATCG GAGAATCAGA
GCAGTAGCTC GGACTCCGAG 240

GCGGAGAGGC GCCCGCAGCC CGCCCGGGAC GCTTTCCAGA
AGCCGCGCGA CTATTTCGCC 300

GAAGTGAGAA GGCCCCAGGA TGGCGCTTTT TTTAAGGGAG
GTGCGTATCC TGGGTACCCC 360

TTCCTGATGA TTCCAGACCT GAGCAGCCCG TACCTCTCCA ACGGACCCCT
GTCTCCTGGT 420

GGAGCGCGCA CCTACCTACA GATGAAATGG CCCCTCCTCG
ATGTCCCCTC CAGTGCTACA    480

GTCAAGGACA CAAGGTCGCC ATCTCCAGCA CACTTGTCCA
ACAAAGTTCC TGTTGTTCAG    540

CACCCTCATC ACATGCACCC GCTGACACCT CTCATCACCT ACAGCAACGA
CCACTTCTCC    600

CCTGCGTCCC CTCCCACGCA TCTGTCCCCA GAGATCGACC
CAAAGACAGG AATCCCCCGG    660

CCCCCTCATC CATCTGAGCT GTCACCGTAT TACCCACTGT CTCCAGGAGC
TGTCGGACAA    720

ATCCCCCATC CCCTCGGCTG GCTCGTCCCA CAGCAAGGGC
AGCCCATGTA CTCTCTCCCT    780

CCGGGTGGTT TCCGGCATCC TTACCCTGCC CTCGCCATGA ACGCTTCAAT
GTCCAGCCTG    840

GTCTCAAGCC GGTTCCCACA CATGGTGGCT CCTGCCCATC CTGGTCTGCC
CACCTCAGGA    900

ATCCCCCACC CTGCCATCGT CTCCCCCATT GTGAAGCAGG
AGCCAGCAGC CCCCAGCCTG    960

AGCCCTGCAG TGAGTGCGAA ATCCCCAGTT ACGGTGAAGA
AGGAAGAGGA GAAGAAACCT    1020

CACGTGAAAA AGCCCCTGAA TGCCTTCATG TTGTATATGA
AGGAGATGAG GGCCAAGGTG    1080

GTGGCCGAGT GTACCCTGAA GGAAAGTGCA GCCATTAACC
AAATCCTGGG AAGAAAGTGG    1140

CACAACCTGT CAAGAGAAGA ACAGGCCAAA TACTATGAGC
TTGCCCGGAA AGAACGGCAG    1200

CTTCACGCGC AGCTCTACCC AACCTGGTCA GCCCGGGACA
ACTATGGGAA GAAGAAGAAG    1260

AGGAAGAGAG AAAAGCAGCT GTCACAGACA CAGTCTCAGC
AGCAAATCCA AGAGGCAGAG    1320

GGTGCTCTGG CCTCTAAGAG CAAGAAGCCA TGCATTCAGT
ACCTGCCCCC TGAGAAGCCT    1380

TGT[?]GATAGCC CTGCGTCTTC CCATGGCAGC GCGCTGGACT
CCCCTGCGAC GCCCTCCGCA    1440

GCCTTGGCCT CACCAGCTGC CCCTGCGGCT ACCCACTCCG
AGCAAGCGCA GCCCCTGTCG    1500

CTCACCACCA AACCGGAGGC CCGGGCCCAG CTGGCTCTCC
ACTCCGCTGC CTTCCTGTCG    1560

GCTAAGGCGG CCGCCAGCAA CTCCAGCCAG ATGGGCAGCC
AGCCCCCACT CCTATCCAGG    1620

CCCTTGCCCT TGGGCTCTAT GCCCGCCGCT CTGCTGACCT CTCCCCCTAC
TTTCCCAGCT    1680

ACGCTCCATG CCCACCAGGC CCTCCCCGTG CTACAGGCCC AGCCTCTTTC
CTTGGTCACC    1740

AAGTCTGCCC ACTAAGCTGC CCCCGGTTCT ATGCAGGCTG
TCTCGGGACT CCAAGTAGTA    1800

ATGATTCAGA GGAGAGGAAG GGGGAAACAC AAAGGAAGCA
TTATTGGTCA ATATTTGACC    1860

ACTCTGGACT GTTCTGTAAA GTGACTGGTA ACAGCAGTGC
CTTACCACTG TAGATATAAC    1920

CAGTAGCTGA TCTTCAGGCT TTTTT                                    1945

(2) INFORMATION FOR SEQ ID NO: 8:

(i) SEQUENCE CHARACTERISTICS:
(A) LENGTH: 2171 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 8:

ATGCCGCAGC TGAACGGCGG TGGAGGAGAT GACCTAGGCG
CTAACGACGA GCTGATCTCC    60

TTCAAAGACG AAGGCGAGCA GGAGGAGAAG AACTCGGAAA
ACTCCTCGGC GGAAAGGGAT 120

TTAGCCGATG TCAAGTCCTC GCTGGTCAAT GAATCAGAGA
CGAATCAAGA CAGCTCCTCC 180

GATTCCGAGG CGGAAAGACG GCCTCCGCCT CGCTCCGAAA
GTTTCCGAGA TAAATCCCGG 240

GAAAGTTTGG AAGAAGCGGC CAAGAGGCAA GATGGAGGGC
TCTTTAAGGG GCCACCGTAT 300

CCCGGCTACC CCTTCATCAT GATCCCCGAC CTGACGAGCC CCTACCTCCC
CAACGGATCG 360

CTCTCGCCCA CCGCCCGAAC CTATCTTCAG ATGAAATGGC CACTGCTTGA
TGTCCAAGCA 420

GGAAGCCTCC AGAGCAGACA AACCCTCAAG GATGCTCGTT
CGCCGTCGCC AGCACACATC 480

GTTTCGAACA AAGTACCGGT GGTGCAACAC CCCCACCATG
TCCACCCACT CACGCCTCTC 540

ATCACGTACA GCAATGAACA CTTCACCCCG GGAAATCCAC
CTCCGCACTT ACCAGCTGAC 600

GTAGACCCCA AAACAGGAAT CCCAAGGCCT CCGCACCCTC
CAGATATCTC TCCATATTAC 660

CCGCTGTCGC CCGGCACCGT AGGACAAATC CCCCATCCGC
TAGGATGGTT AGTACCACAG 720

CAAGGTCAGC CTGTGTACCC AATCACGACA GGAGGATTCA
GACATCCCTA CCCCACAGCG 780

CTGACAGTCA ACGCATCTAT GTCTAGGTTC CCTCCCCATA TGGTCCCTCC
CCATCACACT 840

CTGCACACGA CCGGCATCCC TCACCCGGCC ATCGTCACAC
CGACAGTCAA GCAGGAATCC 900

TCCCAGAGTG ACGTCGGCTC ACTCCACAGC TCAAAGCATC
AGGACTCCAA AAAGGAAGAA 960

GAGAAGAAGA AGCCCCACAT AAAGAAGCCC CTTAATGCAT
TCATGTTGTA TATGAAAGAG 1020

ATGAGAGCGA AGGTGGTGGC CGAATGCACA TTGAAAGAGA
GTGCAGCCAT CAACCAGATT 1080

CTCGGGCGCA GGTGGCACGC CCTGTCCAGG GAAGAACAGG
CAAAATACTA CGAGCTGGCC 1140

CGGAAGGAAC GACAGCTTCA CATGCAGCTG TACCCTGGCT
GGTCTGCACG GGATAACTAT 1200

GGGAAGAAGA AGAAGAGAAA AAGAGACAAG CAGCCGGGGG
AAACCAACGA ACACAGCGAA 1260

TGTTTCCTAA ATCCTTGCCT TTCGCTTCCT CCGATCACAG ACCTGAGCGC
TCCTAAGAAA 1320

TGCCGAGCGC GCTTTGGCCT TGATCAACAG AATAACTGGT
GCGGCCCCTG CAGTCTTTGA 1380

ATTTGGAATA TTATGATGGA GAAAAAAAAA AGTGCGTTCG
CTACATACAA GGTGAAGGCA 1440

GCTGCCTCAG CCCACCCTCT TCAGATCCAA GCTTACTAGA CTCGCCTCCC
CCCTCACCGC 1500

ATCTGCTAGG CTCCCCTCCC CAAGACGCCA AGTCACAGAC
TGAGCAGACC CAGCCGCTCT 1560

CGCTGTCCCT GAAGCCTGAT CCTCTGGCCC ACCTGTCCAT GATGCCTCCG
CCACCTGCGC 1620

TCCTGTTGGC CGAAGCTGCC CACGGCAAGG CCTCTGCCCT CTGTCCCAAT
GGGGCTCTGG 1680

ACCTGCCACC TGCCGCTCTG CAGCCGTCCA TGGTCCCTTC CTCATCGCTC
GCACAACCAT 1740

CAACTTCTTC CTTACATTCC CACAACTCGC TGGCTGGAAC GCAACCCCAG
CCTCTGTCTC 1800

TGGTGACCAG GTCTTTAGAA TAGCTCCGCC TCCTCGTCCT CCATGCCCCT
GCTTGATTGA 1860

AGTGTTTCCT TCTGGTTCTT GGTGCGCCCT TCCCCGCTTG GAAAGCTTTT
GTTTTGTACT 1920

CTTTAATTTT GTGCCGCCGT GGCTACATGA GTTAATGTTT ATGGAGTTCA
TTGGTCAATA 1980

TTTGACCCAT TCTTATTTCA ATTTCTCCTT TTAAATATGT AGATGAGAAA
AACCTCATGA 2040

TTCTACCAAA ATTTTTATCC GAAAAATAAC AATACAGGAC TCTTTCGAGG
CCCTGTAATT 2100

GGAATGAGTC CACTTTAAAT CCTTTAACGA GGATCCATTG
GAGGGCAAGT CTGGTGCCAG 2160

CATCCGCGGT A 2171

(2) INFORMATION FOR SEQ ID NO: 9:

(i) SEQUENCE CHARACTERISTICS:
(A) LENGTH: 1326 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 9:

ATGCCGCAGC TGAACGGCGG TGGAGGGGAT GACCTAGGCG
CCAACGACGA ACTGATTTCC 60

TTCAAAGACG AGGGCGAACA GGAGGAGAAG AGCTCCGAAA
ACTCCTCGGC AGAGAGGGAT 120

TTAGCTGATG TCAAATCGTC TCTAGTCAAT GAATCAGAAA
CGAATCAAAA CAGCTCCTCC 180

GATTCCGAGG CGGAAAGACG GCCTCCGCCT CGCTCCGAAA
GTTTCCGAGA CAAATCCCGG 240

GAAAGTTTGG AAGAAGCGGC CAAGAGGCAA GATGGAGGGC
TCTTTAAGGG GCCACCGTAT 300

CCCGGCTACC CCTTCATCAT GATCCCCGAC CTGACGAGCC CCTACCTCCC
CAAGCGATCC 360

GTCTCGCCCA CCGCCCGAAC CTATCTCCAG ATGAAATGGC
CACTGCTTGA TGTCCAGGCA 420

GGGAGCCTCC AGAGTAGACA AGCCCTCAAG GATGCCCGGT
CCCCATCACC GGCACACATT 480

GTCTCTAACA AAGTGCCAGT GGTGCAGCAC CCTCACCATG
TCCACCCCCT CACGCCTCTT 540

ATCACGTACA GCAATGAACA CTTCACGCCG GGAAACCCAC
CTCCACACTT ACCAGCCGAC 600

GTAGACCCCA AAACAGGAAT CCCACGGCCT CCGCACCCTC
CAGATATATC CCCGTATTAC 660

CCACTATCGC CTGGCACCGT AGGACAAATC CCCCATCCGC
TAGGATGGTT AGTACCACAG 720

CAAGGTCAAC CAGTGTACCC AATCACGACA GGAGGATTCA
GACACCCCTA CCCCACAGCT 780

CTGACCGTCA ATGCTTCCGT GTCCAGGTTC CCTCCCCATA TGGTCCCACC
ACATCATACG 840

CTACACACGA CGGGCATTCC GCATCCGGCC ATAGTCACAC
CAACAGTCAA ACAGGAATCG 900

TCCCAGAGTG ATGTCGGCTC ACTCCATAGT TCAAAGCATC
AGGACTCCAA AAAGGAAGAA 960

GAAAAGAAGA AGCCCCACAT AAAGAAACCT CTTAATGCAT
TCATGTTGTA TATGAAGGAA 1020

ATGAGAGCAA AGGTCGTAGC TGAGTGCACG TTGAAAGAAA
GCGCGGCCAT CAACCAGATC 1080

CTTGGGCGGA GGTGGCATGC ACTGTCCAGA GAAGAGCAAG
CGAAATACTA CGAGCTGGCC 1140

CGGAAGGAGC GACAGCTTCA TATGCAACTG TACCCCGGCT
GGTCCGCGCG GGATAACTAT 1200

GGAAAGAAGA AGAAGAGGAA AAGGGACAAG CAGCCGGGAG
AGACCAATGG AGAAAAAAAA 1260

AGTGCGTTCG CTACATACAA GGTGAAGGCA GCTGCCTCAG
CCCACCCTCT TCAGATGGAA 1320

GCTTAC 1326

(2) INFORMATION FOR SEQ ID NO: 10:

(i) SEQUENCE CHARACTERISTICS:
(A) LENGTH: 1788 base pairs
(B) TYPE: nucleic acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 10:

ATGCCGCAGC TGAACGGCGG TGGAGGGGAT GACCTAGGCG
CCAACGACGA ACTGATTTCC 60

TTCAAAGACG AGGGCGAACA GGAGGAGAAG AGCTCCGAAA
ACTCCTCGGC AGAGAGGGAT 120

TTAGCTGATG TCAAATCGTC TCTAGTCAAT GAATCAGAAA
CGAATCAAAA CAGCTCCTCC 180

GATTCCGAGG CGGAAAGACG GCCTCCGCCT CGCTCCGAAA
GTTTCCGAGA CAAATCCCGG 240

GAAAGTTTGG AAGAAGCGGC CAAGAGGCAA GATGGAGGGC
TCTTTAAGGG GCCACCGTAT 300

CCCGGCTACC CCTTCATCAT GATCCCCGAC CTGACGAGCC CCTACCTCCC
CAAGCGATCC 360

GTCTCGCCCA CCGCCCGAAC CTATCTCCAG ATGAAATGGC
CACTGCTTGA TGTCCAGGCA 420

GGGAGCCTCC AGAGTAGACA AGCCCTCAAG GATGCCCGGT
CCCCATCACC GGCACACATT 480

GTCTCTAACA AAGTGCCAGT GGTGCAGCAC CCTCACCATG
TCCACCCCCT CACGCCTCTT 540

ATCACGTACA GCAATGAACA CTTCACGCCG GGAAACCCAC
CTCCACACTT ACCAGCCGAC 600

GTAGACCCCA AAACAGGAAT CCCACGGCCT CCGCACCCTC
CAGATATATC CCCGTATTAC 660

CCACTATCGC CTGGCACCGT AGGACAAATC CCCCATCCGC
TAGGATGGTT AGTACCACAG 720

CAAGGTCAAC CAGTGTACCC AATCACGACA GGAGGATTCA
GACACCCCTA CCCCACAGCT 780

CTGACCGTCA ATGCTTCCGT GTCCAGGTTC CCTCCCCATA TGGTCCCACC
ACATCATACG 840

CTACACACGA CGGGCATTCC GCATCCGGCC ATAGTCACAC
CAACAGTCAA ACAGGAATCG 900

TCCCAGAGTG ATGTCGGCTC ACTCCATAGT TCAAAGCATC
AGGACTCCAA AAAGGAAGAA 960

GAAAAGAAGA AGCCCCACAT AAAGAAACCT CTTAATGCAT
TCATGTTGTA TATGAAGGAA 1020

ATGAGAGCAA AGGTCGTAGC TGAGTGCACG TTGAAAGAAA
GCGCGGCCAT CAACCAGATC 1080

CTTGGGCGGA GGTGGCATGC ACTGTCCAGA GAAGAGCAAG
CGAAATACTA CGAGCTGGCC 1140

CGGAAGGAGC GACAGCTTCA TATGCAACTG TACCCCGGCT
GGTCCGCGCG GGATAACTAT 1200

GGAAAGAAGA AGAAGAGGAA AAGGGACAAG CAGCCGGGAG
AGACCAATGA ACACAGCGAA 1260

TGTTTCCTAA ATCCTTGCCT TTCACTTCCT CCGATTACAG ACCTCAGCGC
TCCTAAGAAA 1320

TGCCGAGCGC GCTTTGGCCT TGATCAACAG AATAACTGGT
GCGGCCCTTG CAGGAGAAAA 1380

AAAAAGTGCG TTCGCTACAT ACAAGGTGAA GGCAGCTGCC
TCAGCCCACC CTCTTCAGAT 1440

GGAAGCTTAC TAGATTCGCC TCCCCCCTCC CCGAACCTGC TAGGCTCCCC
TCCCCGAGAC 1500

GCCAAGTCAC AGACTGAGCA GACCCAGCCT CTGTCGCTGT
CCCTGAAGCC CGACCCCCTG 1560

GCCCACCTGT CCATGATGCC TCCGCCACCC GCCCTCCTGC TCGCTGAGGC
CACCCACAAG 1620

GCCTCCGCCC TCTGTCCCAA CGGGGCCCTG GACCTGCCCC CAGCCGCTTT
GCAGCCTGCC 1680

GCCCCCTCCT CATCAATTGC ACAGCCGTCG ACTTCTTGGT TACATTCCCA
CAGCTCCCTG 1740

GCCGGGACCC AGCCCCAGCC GCTGTCGCTC GTCACCAAGT CTTTAGAA
1788